# EUROPEAN PATENT SPECIFICATION

(11) **EP 3 598 945 B1**
(45) Date of publication and mention of the grant of the patent: **01.06.2022**
(21) Application number: 17901981.5
(22) Date of filing: 22.12.2017
(51) Int. Cl.: A61B 5/22, A61B 5/0245, A61B 5/11, A61B 5/024, A61B 5/00

(54) **INFORMATION PROCESSING DEVICE, INFORMATION PROCESSING METHOD, AND PROGRAM**
INFORMATIONSVERARBEITUNGSVORRICHTUNG, INFORMATIONSVERARBEITUNGSVERFAHREN UND PROGRAMM
DISPOSITIF DE TRAITEMENT D'INFORMATIONS, PROCÉDÉ DE TRAITEMENT D'INFORMATIONS ET PROGRAMME

(30) Priority: 21.03.2017 JP 2017054056
(43) Date of publication of application: 29.01.2020
(73) Proprietor: Sony Group Corporation, Tokyo 108-0075 (JP)
(72) Inventor: WAKITA, Yoshihiro, Tokyo 108-0075 (JP); SAZUKA, Naoya, Tokyo 108-0075 (JP)
(74) Representative: MFG Patentanwälte Meyer-Wildhagen Meggle-Freund Gerhard PartG mbB
(86) International application number: PCT/JP2017/046266
(87) International publication number: WO 2018/173401

(56) References cited:
- WO-A1-2016/162519
- JP-A- S59 149 128
- JP-A- 2002 330 933
- JP-A- 2005 230 340
- JP-A- 2008 104 758
- US-A1- 2014 172 314

## Description

### TECHNICAL FIELD

The present disclosure relates to an information processing apparatus, an information processing method, and a program.

### BACKGROUND ART

In recent years, a device has been required which can easily grasp energy consumed by daily activity, sports, and the like for health maintenance, physical fitness development, dieting, and the like. As an example of such a device, a device can be exemplified which measures a heart rate or a pulse rate of a user and estimates the consumed energy on the basis of a relation between the heart rate and the like corresponding to an exercise intensity at the time of measurement and the consumed energy. Furthermore, as another example, a device can be exemplified which estimates the consumed energy on the basis of attribute information such as the height, the weight, the age, and the gender, of the user and an exercise intensity obtained by an accelerometer attached to the user or a moved distance of the user. As an example of the latter case, a portable health management device disclosed in Patent Document 1 described below can be mentioned. The device disclosed in the following Patent Document 1 can perform estimation with high accuracy in a case where consumed energy of the user caused by walking is estimated.

From patent application WO 2016/162519 A1, a vital signs monitoring system with a processing unit for estimating an energy expenditure is known.

### CITATION LIST

### PATENT DOCUMENT

Patent Document 1: Japanese Patent Application Laid-Open No. 2002-45352

### SUMMARY OF THE INVENTION

### PROBLEMS TO BE SOLVED BY THE INVENTION

However, according to the device described above, it has been difficult to estimate consumed energy in daily activity which is a series of various exercises in a short time with high accuracy.

Therefore, in the present disclosure, a novel and improved information processing apparatus, information processing method, and program which can estimate consumed energy with high accuracy are proposed.

### SOLUTIONS TO PROBLEMS

According to a first aspect, the invention provides a wearable device in accordance with claim 1. According to a second aspect, the invention provides an information processing method in accordance with claim 6. According to a third aspect, the invention provides a computer program in accordance with claim 7. Further aspects are set forth in the dependent claims, the drawings, and the following description.

According to the present disclosure, an information processing apparatus is provided which includes an acquisition unit that acquires physical characteristics of a user and an estimator based on a relation between a beating rate and consumed energy, in which consumed energy consumed by an activity performed by the user is estimated according to a beating rate of the user by the estimator according to the physical characteristics of the user.

Furthermore, according to the present disclosure, an information processing method is provided which includes acquiring physical characteristics of a user and estimating consumed energy by an activity performed by the user from a beating rate of the user on the basis of a relation between the beating rate and the consumed energy according to the physical characteristics of the user.

Moreover, according to the present disclosure, a program is provided which causes a computer to implement a function for acquiring physical characteristics of a user and a function for estimating consumed energy by an activity performed by the user from a beating rate of the user on the basis of a relation between the beating rate and the consumed energy according to the physical characteristics of the user.

### EFFECTS OF THE INVENTION

As described above, according to the present disclosure, an information processing apparatus, an information processing method, and a program which can estimate consumed energy with high accuracy can be provided.

Note that the above effects are not necessarily limited, and any effect indicated in the present description or other effect which can be recognized from the present description may be obtained together with or instead of the above effects.

### BRIEF DESCRIPTION OF DRAWINGS

Fig. 1 is an explanatory diagram for explaining an exemplary configuration of an information processing system 1 according to a first embodiment of the present disclosure.
Fig. 2 is a block diagram illustrating a configuration of a wearable device 10 according to the first embodiment.
Fig. 3 is an explanatory diagram illustrating an example of an appearance of the wearable device 10 according to the first embodiment.
Fig. 4 is an explanatory diagram illustrating another example of the appearance of the wearable device 10 according to the first embodiment.
Fig. 5 is an explanatory diagram illustrating an example of a wearing state of the wearable device 10 according to the first embodiment.
Fig. 6 is a block diagram illustrating a configuration of a server 30 according to the first embodiment.
Fig. 7 is a block diagram illustrating a configuration of a user terminal 50 according to the first embodiment.
Fig. 8 is an explanatory diagram illustrating an example of an appearance and a use form of the user terminal 50 according to the first embodiment.
Fig. 9 is an explanatory diagram (No. 1) for explaining an operation of a learning unit 132 according to the first embodiment.
Fig. 10 is an explanatory diagram (No. 2) for explaining the operation of the learning unit 132 according to the first embodiment.
Fig. 11 is an explanatory diagram (No. 1) for explaining an operation of a likelihood estimator 236 according to the first embodiment.
Fig. 12 is an explanatory diagram (No. 2) for explaining the operation of the likelihood estimator 236 according to the first embodiment.
Fig. 13 is an explanatory diagram (No. 3) for explaining the operation of the likelihood estimator 236 according to the first embodiment.
Fig. 14 is an explanatory diagram for explaining an operation of an estimation unit 136 according to the first embodiment.
Fig. 15 is a flow diagram for explaining an example of a learning stage in an information processing method according to the first embodiment.
Fig. 16 is a flow diagram for explaining an example of an estimation stage in the information processing method according to the first embodiment.
Fig. 17 is an explanatory diagram for explaining an example of a display screen 800 used when time-series data is acquired.
Fig. 18 is an explanatory diagram for explaining an example of a display screen 802 used when the time-series data is acquired.
Fig. 19 is an explanatory diagram for explaining an example of a display screen 804 used when the time-series data is acquired.
Fig. 20 is an explanatory diagram for explaining an example of a display screen 806 used when the time-series data is acquired.
Fig. 21 is an explanatory diagram for explaining a method for leading a user to exercise when the time-series data is acquired.
Fig. 22 is an explanatory diagram for explaining an example of a display screen 808 used when time-series data is acquired by another method.
Fig. 23 is an explanatory diagram for explaining an example of a display screen 810 used when time-series data is acquired by still another method.
Fig. 24 is an explanatory diagram for explaining an example of a display screen 812 used when time-series data is acquired by yet another method.
Fig. 25 is an explanatory diagram for explaining an example of a display screen 814 used when time-series data is acquired by still yet another method.
Fig. 26 is an explanatory diagram for explaining another method for leading the user to exercise when the time-series data is acquired.
Fig. 27 is an explanatory diagram for explaining an example of a display screen 820 according to an example 1.
Fig. 28 is an explanatory diagram for explaining an example of a display screen 822 according to the example 1.
Fig. 29 is an explanatory diagram for explaining an example of a display screen 824 according to an example 2.
Fig. 30 is an explanatory diagram for explaining an example of a display screen 826 according to the example 2.
Fig. 31 is an explanatory diagram for explaining an example of a display screen 828 according to the example 2.
Fig. 32 is an explanatory diagram for explaining an example of a display screen 830 according to the example 2.
Fig. 33 is an explanatory diagram illustrating a temporal change 90 of an actual measurement value of consumed energy and a temporal change 92 of a heart rate obtained by study of the present inventors.
Fig. 34 is a block diagram illustrating an exemplary hardware configuration of an information processing apparatus 900 according to one embodiment of the present disclosure.

### MODE FOR CARRYING OUT THE INVENTION

Hereinafter, a preferred embodiment of the present disclosure will be described in detail with reference to the accompanying drawings. Note that, in the present description and the drawings, components having substantially the same functional configurations are denoted with the same reference numeral so as to omit redundant description.

Furthermore, in the present description and the drawings, there is a case where a plurality of components having substantially the same or similar functional configuration is distinguished from each other by attaching different numerals after the same reference numerals. However, in a case where it is not necessary to particularly distinguish the plurality of components having substantially the same or similar functional configuration from each other, only the same reference numeral is applied. Furthermore, there is a case where components similar to each other in different embodiments are distinguished from each other by adding different alphabets after the same reference numeral. However, in a case where it is not particularly necessary to distinguish the plurality of similar components from each other, only the same reference numeral is applied.

Note that, in the description below, a "beating rate" includes a heart rate and a pulse rate, and it is assumed that the heart rate and the pulse rate can be respectively measured by a heart rate sensor and a pulse rate sensor.

Furthermore, the description will be made in the following order.
1. History before embodiment of present disclosure is created
2. Embodiment according to present disclosure
2.1. Outline of information processing system 1 according to present embodiment
2.2. Configuration of wearable device 10 according to present embodiment
2.3. Configuration of server 30 according to present embodiment
2.4. Configuration of user terminal 50 according to present embodiment
2.5. Configuration of control unit 130 according to present embodiment
2.6. Information processing method according to present embodiment
3. Examples according to present embodiment
3.1. Example 1
3.2. Example 2
4. Summary
5. Regarding hardware configuration
6. Supplement

### <<1. History before embodiment of present disclosure is created>>

First, before explaining an embodiment according to the present disclosure, the history before the present inventors have created the embodiment of the present disclosure, in other words, study made by the present inventors will be described.

The present inventors have examined about a method for estimating consumed energy caused by an activity of a user. First, a method for measuring the consumed energy will be described. Methods for measuring the consumed energy mainly include two kinds of measurement methods including a "direct calorimetric method" for directly measuring generated heat and an "indirect calorimetric method" for indirectly measuring calories from a consumed amount of oxygen used in the body.

Since the consumed energy consumed in the human body is dissipated as heat from the human body, in the "direct calorimetric method", the consumed energy is measured by directly measuring the dissipated heat amount from the human body. However, a measurement device used in the "direct calorimetric method" is extremely large and limits an activity of a subject to be measured. Therefore, a situation in which the consumed energy can be measured by the "direct calorimetric method" is limited.

On the other hand, the "indirect calorimetric method" measures an oxygen concentration and a carbon dioxide concentration in breath of the user and calculates the consumed energy from the measurement results. Generation of energy in the human body is caused by decomposing fat and sugar taken from food and the like, and in many cases, oxygen taken in the human body by breathing is needed to perform such decomposition. Therefore, the consumed amount of oxygen approximately corresponds to the consumed energy. Moreover, carbon dioxide is generated by such decomposition in the human body, and the generated carbon dioxide is discharged as a part of the breath from the human body. Therefore, the oxygen concentration and the carbon dioxide concentration in the breath are measured and the consumed amount of oxygen in the human body of the user is obtained from these measurement values so as to recognize the consumed energy. In other words, although the "indirect calorimetric method" is not a method for directly measuring calories generated in the human body, it is possible to almost correctly grasp the consumed energy by a metabolic mechanism in the above-described human body. Since a respiration measuring device used in the "indirect calorimetric method" is simpler than the device used in the above-described "direct calorimetric method", the "indirect calorimetric method" is used in general as the method for measuring the consumed energy. Note that, in the following description, it is assumed that an actual measurement value of the consumed energy be measured by the above-described "indirect calorimetric method".

However, in the "indirect calorimetric method", it is necessary to measure the oxygen concentration and the carbon dioxide concentration in the breath. Therefore, the subject wears a mask and the like. Therefore, although the "indirect calorimetric method" is simpler than the "direct calorimetric method", it is difficult for a general user to easily measure the consumed energy. Therefore, the present inventors have diligently studied about the method for recognizing the consumed energy on the basis of an index which can be easily measured. Note that, here, the general user means a person who is not an expert such as a researcher, a doctor, or an athlete.

On the basis of such a situation, the present inventors have repeatedly studied about a method for recognizing the consumed energy by using a beating rate (specifically, heart rate or pulse rate) which is generally said to be highly correlated with the consumed energy. Specifically, although metabolic activities are performed to supplement the consumed energy in each part of the body, oxygen is consumed and carbon dioxide is generated at that time. Although the consumed amount of oxygen and the generated amount of carbon dioxide are not completely proportional to each other, it is known that both amounts have a relation having monotonicity substantially close to the proportion. The heartbeat means that muscles of the heart contract and expand at a constant rhythm so as to beat, and the heartbeat makes blood be supplied throughout the body through arteries so that oxygen required for metabolism is supplied to each organ in the body. Furthermore, carbon dioxide generated by metabolism elutes in the blood and is collected in the heart via veins, and is discharged to the breath by lung gas exchange. It is known that the numerical value of the heart rate fluctuates depending on the need to enhance blood circulation in the body. However, regarding the relation with energy consumption, it is known that the heart rate is controlled by a mechanism linked to a blood carbon dioxide level. In other words, the heart rate has the property of which the numerical value fluctuates according to the need for carbon dioxide discharge, which is one of needs for enhancing the blood circulation in the body. Therefore, the heart rate has a strong correlation with the consumed energy via the generation amount of carbon dioxide and is useful information for estimating the consumed energy. Note that, in the following description, the heart rate means the number of beats of the heart per unit time, and the pulse rate indicates the number of times of beats of the arteries, appearing on a body surface and the like per unit time, caused by the heartbeat supplying blood in the whole body through the arteries and changing a pressure on an inner surface of the artery.

Furthermore, in recent years, sizes of the heart rate sensor for measuring the heart rate and the pulse rate sensor for measuring the pulse rate are reduced, and it is possible to wear the heart rate sensor and the pulse rate sensor on the body of the user and measure the heart rate or the pulse rate of the user without limiting the activities of the user. For such reasons, the present inventors have studied about a method for recognizing the consumed energy by using the beating rate (heart rate or pulse rate).

Specifically, a method which is examined first by the present inventors measures the heart rate of the user by the heart rate sensor and estimates the consumed energy by using linear regression and the like on the basis of the measured heart rate. According to the study of the present inventors, it is found that, in the method described above, for example, since the heart rate changes according to the exercise intensity in a case where consumed energy in training using a cardio-type training machine is estimated, high estimation accuracy can be obtained. However, according to the method described above, it is found that the estimation accuracy is lowered in a case where the consumed energy at rest is estimated. This is considered because the heart rate largely fluctuates due to not only the exercise intensity but also psychological factors such as tension and excitement.

Therefore, the present inventors have studied the method obtained by improving the above method which is a method for estimating the consumed energy by using not only the heart rate measured by the heart rate sensor but also the exercise intensity detected by the acceleration sensor. Specifically, one of methods studied by the present inventors is a method for adjusting a contribution rate of the exercise intensity in a linear regression equation for estimating the consumed energy from the heart rate, on the basis of the detected exercise intensity. In this method, it is attempted to improve the estimation accuracy of the consumed energy by adjusting the contribution rate. Furthermore, another one of the studied methods is a method for classifying current activity states of the user into several kinds of activity patterns such as a resting state, a walking state, and a running state on the basis of the exercise intensity detected by the acceleration sensor and switching the regression equation to be used according to the classified activity pattern. In this method, in a case where limitation in the estimation by using a predetermined regression equation occurs, it is attempted to improve the estimation accuracy of the consumed energy by using another regression equation optimal for this state.

However, when the present inventors have studied about the above method, it is found that there is a limitation in the improvement in the estimation accuracy of the consumed energy. More specifically, the consumed energy estimated from the heart rate by using the above method tends to be higher than the actual consumed energy according to the activity content of the user and the like, and it is found that it is difficult to improve the estimation accuracy according to such tendency. Moreover, in a case where total consumed energy in one day of one user is estimated by the above method, sufficient estimation accuracy can be obtained. However, in a case where the consumed energy in the individual exercise in a short time is estimated, sufficient estimation accuracy cannot be obtained.

This is because the relation between the heart rate of the user and the consumed energy at a certain point of time is determined not only on the basis of the exercise intensity or the activity state of the user at that point of time and fluctuates as being affected by the previous activity content of the user that point of time according to physical characteristics of the user. In the method described above, since the regression equation is selected according to the exercise intensity or the like at a certain point of time, although the exercise intensity at that point of time is considered, the effect of the previous activity content of the user that point of time according to the physical characteristics of the user is not considered. As a result, in the method, the consumed energy estimated on the basis of the heart rate tends to be higher than the actual consumed energy, and the improvement in the estimation accuracy is limited. In the following description, the relation between the heart rate of the user and the consumed energy fluctuates by being affected from the previous activity content of the user according to the physical characteristics of the user will be described in detail.

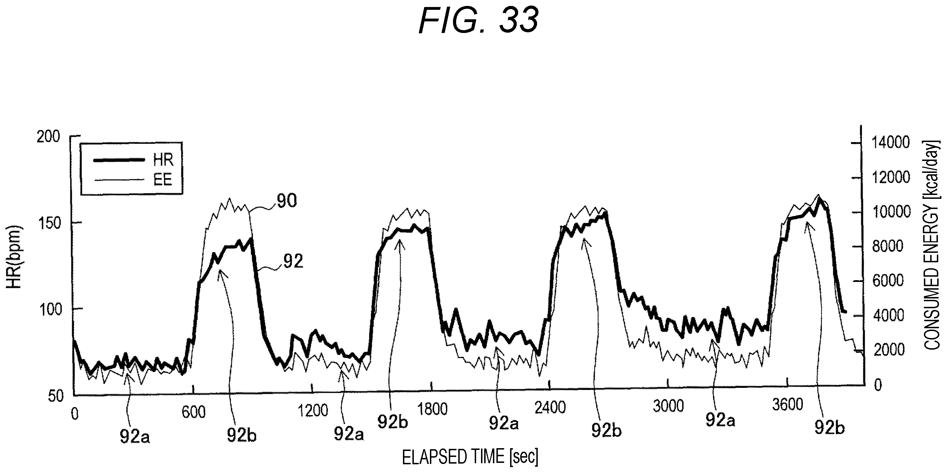

The present inventors make the subject intermittently perform an exercise with a predetermined exercise intensity and measure the consumed energy and the heart rate of the subject by using the indirect calorimetric method. In Fig. 33, a temporal change 90 in the consumed energy and a temporal change 92 in the heart rate obtained by the measurement above are illustrated. As illustrated in Fig. 33, it is found that, although the temporal change 90 in the consumed energy increases to a specific value by the exercise performed by the subject, the temporal change 90 falls to a value almost the same as a value in the resting state before the start of the exercise when the exercise is terminated. On the other hand, similar to the temporal change 90 in the consumed energy, the temporal change 92 in the heart rate increases by the exercise and decreases as the exercise is terminated. However, the temporal change 92 in the heart rate is different from the temporal change 90 in the consumed energy, and the temporal change 92 does not fall to the value almost the same as that in the resting state before the start of the exercise although the temporal change 92 falls when the exercise is terminated. Furthermore, although the temporal change 92 in the heart rate similarly changes to the temporal change 92 in the consumed energy at the time of first exercise, the temporal change 92 gradually increases as the subject repeats the exercise. More specifically, as illustrated in Fig. 33, a temporal change in the heart rate corresponding to a period in which the exercise is stopped (section 92a projecting downward in Fig. 33) increases as the subject repeats the exercise. Furthermore, a temporal change in the heart rate corresponding to a period in which the exercise is performed (section 92b projecting upward in Fig. 33) increases as the subject repeats the exercise. In other words, it has been obvious that there is a case where the fluctuation in the heart rate is separated from the fluctuation in the consumed energy. In other words, according to the study of the present inventors, it is found that, although the relation between the heart rate and the consumed energy can be expressed by a regression equation before the separation is made, in a case where the separation is made, the relation cannot be expressed by the regression equation described above. Note that, in the following description, the separation phenomenon is referred to an enhancement phenomenon in the heart rate since only the heart rate increases. In a case where such an enhancement phenomenon of the heart rate appears, consumed energy estimated by inputting the heart rate into the regression equation is higher than the actual consumed energy.

Moreover, when the present inventors repeatedly perform the measurement as described above on the plurality of subjects, it is confirmed that the enhancement phenomenon is reproducible. In addition, it is confirmed that the appearance pattern of the enhancement phenomenon has different tendency for each subject. Specifically, when the measurement is repeatedly performed on the same subject, it is confirmed that the enhancement phenomenon appears as being reproducible in a case where the exercise exceeds a certain threshold (specifically, in a case where exercise intensity of performed exercise exceeds certain threshold, in case where the number of times of exercises exceeds certain threshold, in a case where exercise time of the performed exercise exceeds certain threshold, and the like). Furthermore, when the same measurement is performed on the different subjects, it is confirmed that the appearance pattern of the enhancement phenomenon differs for each subject. For example, the appearance pattern of the enhancement phenomenon includes a case where the enhancement phenomenon appears only in a period of high exercise intensity, a case where the enhancement phenomenon appears in a period of low exercise intensity such as at the time of resting, a case where the enhancement phenomenon appears in both the high exercise intensity period and the low exercise intensity period, a case where the enhancement phenomenon does not appear, and the like. Furthermore, it is also confirmed that a time constant at the time when the enhancement phenomenon is reduced with time after the exercise is terminated is different for each subject. Moreover, when a large number of measurement results obtained by the present inventors are scrutinized, it is found that the appearance patterns of the enhancement phenomenon can be classified into several groups according to the tendency of the appearance pattern of the enhancement phenomenon.

By the way, as a phenomenon in which the heart rate is not immediately lowered when the exercise is terminated and the user shifts to the resting state, a phenomenon referred to as "expiration debt" is known. Specifically, in a case where the exercise is performed, an energy amount per unit time consumed by the human body increases, and an amount of metabolism increases so as to supply the increased consumed energy. Although the increase in the amount of metabolism is normally compensated by metabolism using oxygen, in a case where the oxygen supply is not sufficient due to limitation in a cardiopulmonary function, the energy is compensated by metabolism without using oxygen. The metabolism for supplying energy without using oxygen in this way is referred to as "expiration debt" or "oxygen debt". This is because, since the metabolism without using oxygen accumulates products such as lactic acid in the body, it is necessary to metabolize the products by using oxygen later, in other words, the metabolism without using oxygen corresponds to consuming future oxygen supply as a loan. As described above, when "expiration debt" occurs, the substances such as lactic acid are accumulated. Therefore, it is necessary to perform metabolism by using oxygen after the exercise, and the consumed amount of oxygen and the generation amount of carbon dioxide do not return to the standard at the time of rest, decrease in the heart rate is prevented. In this way, the "expiration debt" is a phenomenon for preventing the decrease in the heart rate after the exercise. However, the present inventors assume that the enhancement phenomenon is not caused by the "expiration debt". Specifically, the metabolism of the substance such as lactic acid generated by the "expiration debt" occurs together with the consumption of oxygen and the generation of carbon dioxide, and an increase in heat consumption is observed by the "indirect calorimetric method". In other words, elimination of the "expiration debt" is a phenomenon for increasing both the consumed energy and the heart rate, and a state cannot be described in which the heart rate is enhanced although the consumed energy falls to the resting level. In the enhancement phenomenon, it is considered that the heart rate is enhanced due to a factor such as "expiration debt" in which a model on exercise physiology is not established. A purpose of the present disclosure is to avoid generation of an error in estimation of consumed energy caused by a phenomenon of which a mechanism is not clarified. The enhancement phenomenon is a part of an increase factor of the heart rate excluding factors caused by carbon dioxide. Since the heart rate is adjusted to adjust a blood flow, it is estimated that the increase factor of the enhancement phenomenon is performed to maintain some adaptation in the body. Therefore, it is considered that the tendency of the enhancement phenomenon is determined according to various physical characteristics such as a cardiopulmonary function. For example, in a case where heat discharge is considered, it is considered that the need to increase the blood amount for heat transportation increases the heart rate. However, an increase amount of the heart rate and a duration of the increase in the heart rate are affected by a cardiopulmonary function and a sweating function. Specifically, if the cardiopulmonary function and the sweating function are enhanced, a hear discharge efficiency is increased. Therefore, it is considered that the increase amount of the heart rate is reduced and the duration is shortened. From such characteristics, in a case where the physical characteristics are improved by training, it is estimated that the enhancement phenomenon is generally suppressed. Furthermore, in a case where the physical characteristics are temporarily deteriorated due to a bad physical condition, it is estimated that the enhancement phenomenon more strongly appears than the normal time.

In other words, according to the study of the present inventors, it is found that the relation between the heart rate and the consumed energy can be expressed by a predetermined regression equation in a certain range and cannot be expressed by the regression equation in a range where the enhancement phenomenon of the heart rate appears. In other words, according to the study, it is found that the relation between the heart rate and the consumed energy fluctuates. Moreover, it is found that the fluctuation is caused by an effect of the previous activity content of the user according to the appearance pattern of the enhancement phenomenon of the heart rate specific for the user, in other words, the physical characteristics of the user. Therefore, although the method examined by the present inventors so far considers the exercise intensity at a certain point of time, the effect of the activity content of the user before the point of time according to the physical characteristics of the user is not considered. Therefore, it has been difficult to improve the estimation accuracy.

Therefore, the present inventors have created the embodiment of the present disclosure which can estimate the consumed energy with high accuracy even from the heart rate by considering the above recognition as a single focus point. In other words, according to the embodiment of the present disclosure described below, it is possible to estimate the consumed energy with high accuracy by considering the appearance pattern of the enhancement phenomenon of the heart rate uniquely recognized by the present inventors. For example, in the present embodiment, when a heart rate higher than the heart rate at the time of resting is detected after the exercise with high exercise intensity is performed, in the present embodiment, the consumed energy is estimated after contribution of the effect of the enhancement phenomenon on the heart rate is accurately grasped and the contribution is removed. Hereinafter, an information processing apparatus and an information processing method of this sort according to the embodiment of the present disclosure will be sequentially described in detail.

### <<2. Embodiment according to present disclosure>>

### <2.1. Outline of information processing system 1 according to present embodiment>

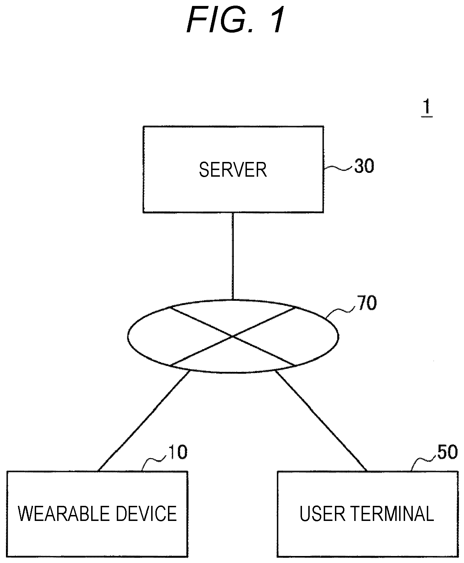

Next, a configuration according to an embodiment of the present disclosure will be described. First, the configuration of the embodiment according to the present disclosure will be described with reference to Fig. 1. Fig. 1 is an explanatory diagram for explaining an exemplary configuration of an information processing system 1 according to the present embodiment.

As illustrated in Fig. 1, the information processing system 1 according to the present embodiment includes a wearable device 10, a server 30, and a user terminal 50 which are communicably connected to each other via a network 70. Specifically, the wearable device 10, the server 30, and the user terminal 50 are connected to the network 70 via a base station and the like (for example, base station of mobile phones, access point of wireless LAN, and the like) which is not illustrated. Note that any method can be applied as a communication method used in the network 70 regardless whether the method is a wired or wireless method. However, it is desirable to use a communication method which can maintain a stable operation.

The wearable device 10 can be a device which can be attached to a part of a body of a user or an implant device (implant terminal) inserted into the body of the user. More specifically, as the wearable device 10, various types of wearable devices can be employed such as a head mounted display (HMD) type, an ear device type, an anklet type, a bracelet type, a collar type, an eyewear type, a pad type, a badge type, and a cloth type. Moreover, the wearable device 10 incorporates sensors such as a heart rate sensor which detects a heart rate of a user (or pulse rate sensor which detects pulse rate of a user), and an acceleration sensor which detects exercise intensity according to an exercise of a user. Note that the wearable device 10 will be described later in detail.

The server 30 is, for example, configured of a computer and the like. The server 30, for example, stores information used in the present embodiment and distributes information provided in the present embodiment. Note that the server 30 will be described later in detail.

The user terminal 50 is a terminal for outputting the information provided in the present embodiment to a user and the like. For example, the user terminal 50 can be a device such as a tablet-type personal computer (PC), a smartphone, a mobile phone, a laptoptype PC, a notebook PC, and an HMD.

Note that, in Fig. 1, the information processing system 1 according to the present embodiment is illustrated as an information processing system 1 including the single wearable device 10 and the single user terminal 50. However, the configuration of the information processing system 1 is not limited to this in the present embodiment. For example, the information processing system 1 according to the present embodiment may include the plurality of wearable devices 10 and the plurality of user terminals 50. Moreover, the information processing system 1 according to the present embodiment may include, for example, another communication device such as a relay device which is used when information is transmitted from the wearable device 10 to the server 30 and the like. Furthermore, in the present embodiment, the wearable device 10 may be used as a stand-alone device. In this case, at least a part of functions of the server 30 and the user terminal 50 is performed by the wearable device 10.

### <2.2. Configuration of wearable device 10 according to present embodiment>

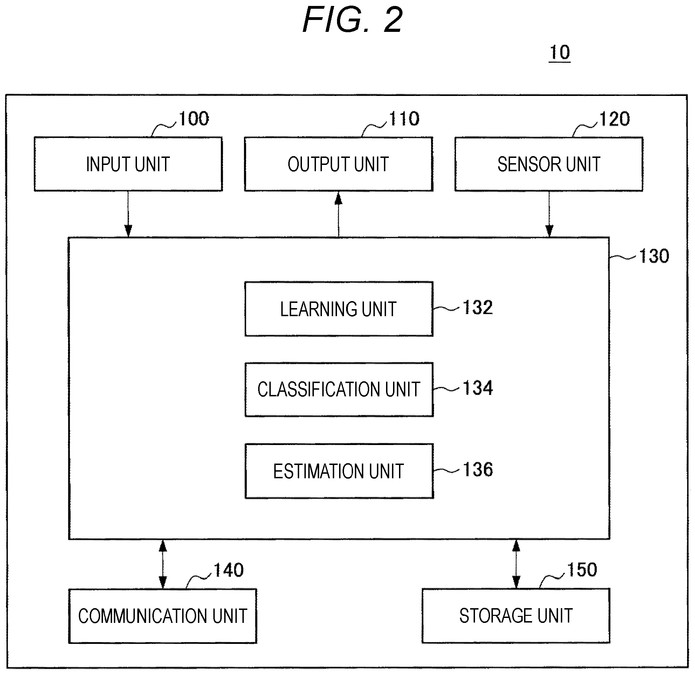

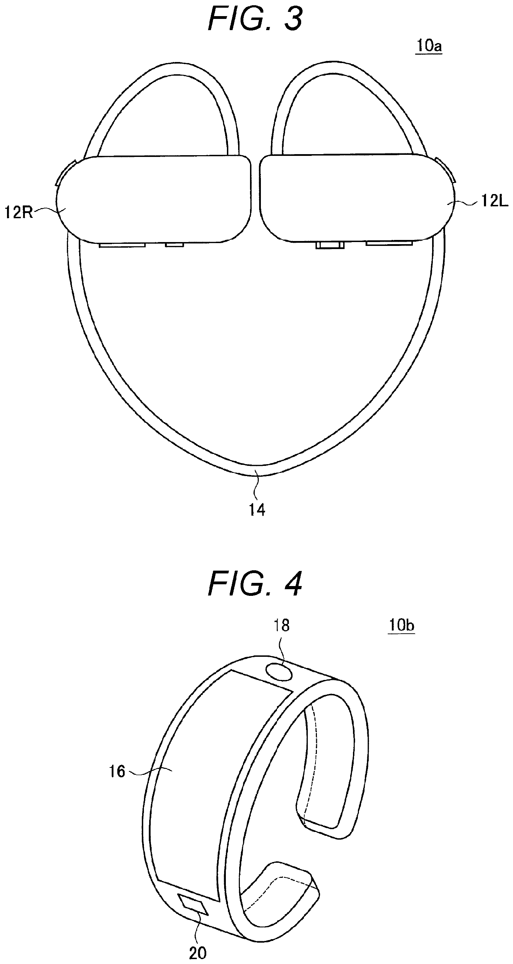

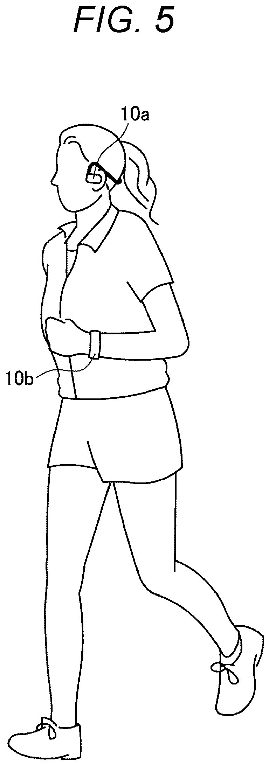

Next, a configuration of the wearable device 10 according to the embodiment of the present disclosure will be described with reference to Figs. 2 to 5. Fig. 2 is block diagram illustrating the configuration of the wearable device 10 according to the present embodiment. Figs. 3 and 4 are explanatory diagrams illustrating an example of an appearance of the wearable device 10 according to the present embodiment. Fig. 5 is an explanatory diagram illustrating an example of a wearing state of the wearable device 10 according to the present embodiment.

As illustrated in Fig. 2, the wearable device 10 mainly includes an input unit (acquisition unit) 100, an output unit 110, a sensor unit (acquisition unit) 120, a control unit 130, a communication unit 140, and a storage unit 150. Each functional unit of the wearable device 10 will be described in detail below.

### (Input unit 100)

The input unit 100 receives an input of data and a command to the wearable device 10. More specifically, the input unit 100 is realized by a touch panel, a button, a microphone, a drive, and the like. For example, the input unit 100 receives information to be used for learning by a learning unit 132 as described later (cluster information, heart rate fluctuation pattern data, consumed energy fluctuation pattern data, and the like) and information to be used for classification and estimation by a classification unit 134 and an estimation unit 136 as described later (cluster information, heart rate fluctuation pattern data, consumed energy fluctuation pattern data, and the like).

### (Output unit 110)

The output unit 110 is a device for presenting information to a user and outputs various information to the user, for example, by an image, voice, light, vibration, or the like. Specifically, to acquire the heart rate fluctuation pattern in a change in a predetermined exercise intensity, the output unit 110 outputs a screen and voice to prompt a user to perform predetermined exercise as an instruction unit. Furthermore, the output unit 110 outputs the information regarding the consumed energy estimated by the control unit 130 as described later and information used to recommend the predetermined exercise on the basis of the estimated consumed energy as a notification unit. The output unit 110 is realized by a display, a speaker, an earphone, a light emitting element, a vibration module, and the like. Note that a function of the output unit 110 may be provided by an output unit 510 of the user terminal 50 as described later.

### (Sensor unit 120)

The sensor unit 120 is provided in the wearable device 10 attached to the body of the user and includes a heart rate sensor (heart rate meter) which detects a heart rate of the user. The heart rate sensor measures the heart rate of the user and outputs the measured result to the control unit 130 as described later. Note that the heart rate sensor may be a pulse rate sensor (pulsometer) which measures a pulse rate of the user. Furthermore, the sensor unit 120 may include a motion sensor for detecting the exercise intensity of the user. The motion sensor includes at least an acceleration sensor (accelerometer), detects a change in the acceleration caused according to an operation of the user, and outputs the detection result to the control unit 130 as described later. Note that, in the embodiment described below, the acceleration change generated according to the operation of the user is used as an index indicating the exercise intensity. The acceleration can be measured by the acceleration sensor, and the acceleration sensor can be easily used even by ordinary people. Therefore, here, the acceleration change is used as the index indicating the exercise intensity. In other words, the sensor unit 120 acquires the information to be used for learning by the learning unit 132 as described later (heart rate fluctuation pattern data and the like) and the information used for classification and estimation by the classification unit 134 and the estimation unit 136 as described later (heart rate fluctuation pattern data, exercise intensity, and the like).

Note that the motion sensor may include a gyro sensor, a geomagnetic sensor, and the like. Moreover, the sensor unit 120 may include various other sensors such as a global positioning system (GPS) receiver, an atmospheric pressure sensor, a temperature sensor, and a humidity sensor. Moreover, the sensor unit 120 may have a built-in clock mechanism (not illustrated) which grasps accurate time and associate the time when the heart rate and the like is acquired with the acquired heart rate, acceleration change, and the like.

### (Control unit 130)

The control unit 130 is provided in the wearable device 10 and can control each block of the wearable device 10 and perform calculation or the like by using the heart rate and the acceleration change output from the sensor unit 120 described above. The control unit 130 is realized by hardware, for example, a central processing unit (CPU), a read only memory (ROM), a random access memory (RAM), and the like. Note that a function of the control unit 130 may be provided by a control unit 330 of the server 30 or a control unit 530 of the user terminal 50 as described later.

Moreover, the control unit 130 can function as the learning unit (learning device) 132, the classification unit 134, and the estimation unit 136, in other words, can estimate the consumed energy on the basis of the relation between the heart rate and the consumed energy, perform classification and learning for estimation. Note that these functional units of the control unit 130 will be described later in detail.

### (Communication unit 140)

The communication unit 140 is provided in the wearable device 10 and can exchange information with an external device such as the server 30 and the user terminal 50. In other words, it can be said that the communication unit 140 is a communication interface having a function for exchanging data. Note that the communication unit 140 is realized by a communication device such as a communication antenna, a transmission and reception circuit, and a port.

### (Storage unit 150)

The storage unit 150 is provided in the wearable device 10 and stores a program, information, and the like used to execute various processing by the control unit 130 described above and information obtained by the processing. Note that the storage unit 150 is realized by, for example, a nonvolatile memory such as a flash memory and the like.

Note that, in the present embodiment, two sensors in the sensor unit 120, i.e., the heart rate sensor and the motion sensor may be provided in different wearable devices 10. Since the configuration of each wearable device 10 can be made compact in this way, the wearable device 10 can be attached to various parts of the body of the user.

As described above, as the wearable device 10, various types of wearable devices such as an eyewear type, an ear device type, a bracelet type, and an HMD type can be employed. In Fig. 3, an example of an appearance of the wearable device 10 is illustrated. A wearable device 10a illustrated in Fig. 3 is an ear device type wearable device which is attached to both ears of the user. The wearable device 10a mainly includes left and right main body portions 12L and 12R and a neck band 14 which connects the main body portions 12L and 12R. The main body portions 12L and 12R incorporate at least a part of, for example, the input unit 100, the output unit 110, the sensor unit 120, the control unit 130, the communication unit 140, and the storage unit 150 illustrated in Fig. 3. Furthermore, each of the main body portions 12L and 12R has an built-in earphone (not illustrated) which functions as the output unit 110, and the user can listen to voice information and the like by wearing the earphones in both ears.

Moreover, another example of the appearance of the wearable device 10 is illustrated in Fig. 4. A wearable device 10b illustrated in Fig. 4 is a bracelet type wearable device. The wearable device 10b is a wearable terminal attached to an arm and a wrist of the user and is referred to as a watch type wearable device. On an outer peripheral surface of the wearable device 10b, a touch panel display 16 having functions as the input unit 100 and the output unit 110 in Fig. 3 is provided. Moreover, on the outer peripheral surface, a speaker 18 having a voice output function as the output unit 110 and a microphone 20 having a sound collecting function as the input unit 100 are provided.

Furthermore, as illustrated in Fig. 5, the single or plurality of wearable devices 10 can be attached to various parts of the user such as a head and a wrist.

### <2.3. Configuration of server 30 according to present embodiment>

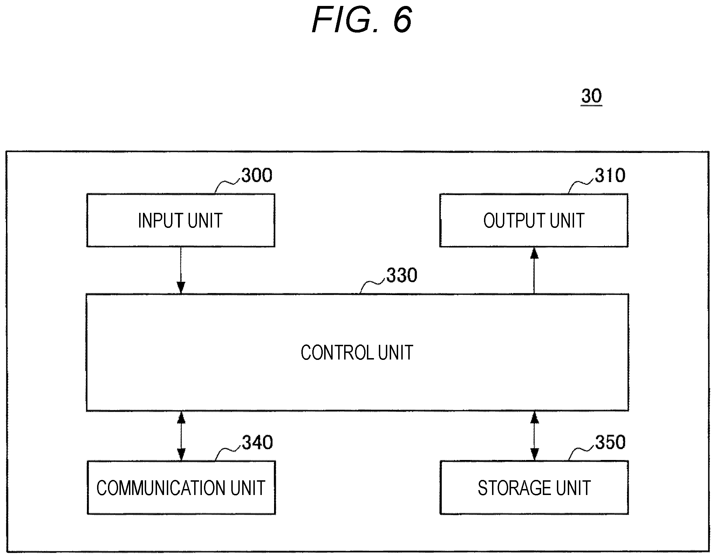

Next, a configuration of the server 30 according to the embodiment of the present disclosure will be described with reference to Fig. 6. Fig. 6 is a block diagram illustrating the configuration of the server 30 according to the present embodiment.

As described above, the server 30 is, for example, configured of a computer and the like. As illustrated in Fig. 6, the server 30 mainly includes an input unit 300, an output unit 310, the control unit 330, a communication unit 340, and a storage unit 350. Each functional unit of the server 30 will be described in detail below.

### (Input unit 300)

The input unit 300 receives an input of data and a command to the server 30. More specifically, the input unit 300 is realized by a touch panel, a keyboard, and the like.

### (Output unit 310)

The output unit 310 includes, for example, a display, a speaker, a video output terminal, a voice output terminal, and the like and outputs various information by an image, voice, or the like.

### (Control unit 330)

The control unit 330 is provided in the server 30 and can control each block of the server 30. The control unit 330 is realized by hardware, for example, a CPU, a ROM, a RAM, and the like. Note that the control unit 330 may execute a part of the function of the control unit 130 of the wearable device 10.

### (Communication unit 340)

The communication unit 340 is provided in the server 30 and can exchange information with an external device such as the wearable device 10 and the user terminal 50. Note that the communication unit 340 is realized by a communication device such as a communication antenna, a transmission and reception circuit, and a port.

### (Storage unit 350)

The storage unit 350 is provided in the server 30 and stores a program and the like used to execute various processing by the control unit 320 described above and information obtained by the processing. More specifically, the storage unit 350 can store data such as the heart rate and the consumed energy obtained from the wearable devices 10 attached to the plurality of users, map data provided to each user, data used for estimation of the consumed energy, and the like. Note that the storage unit 350 is realized by, for example, a magnetic recording medium such as a hard disk (HD), a nonvolatile memory, and the like.

### <2.4. Configuration of user terminal 50 according to present embodiment>

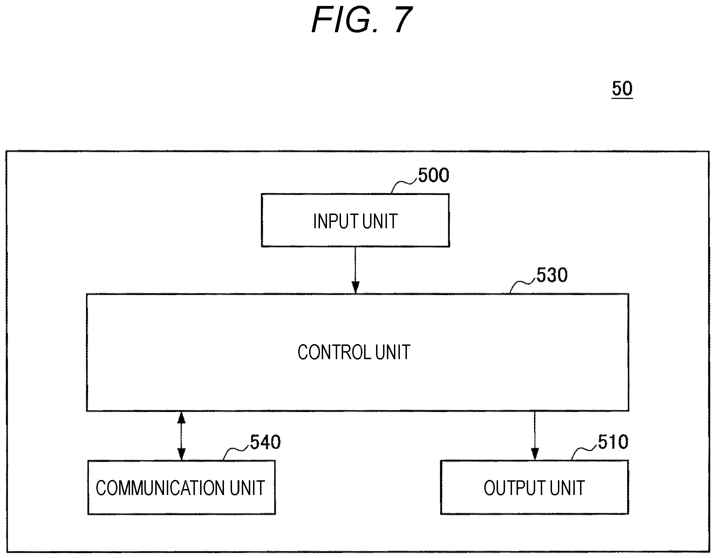

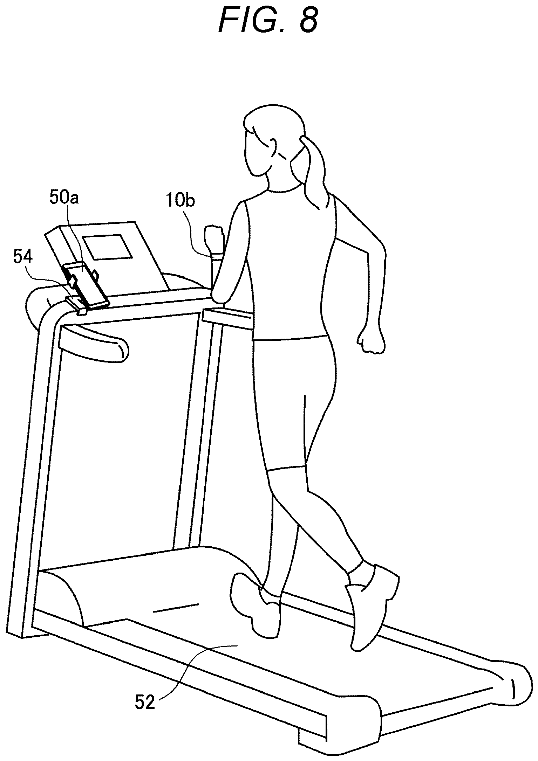

Next, a configuration of the user terminal 50 according to the embodiment of the present disclosure will be described with reference to Figs. 7 and 8. Fig. 7 is a block diagram illustrating the configuration of the user terminal 50 according to the present embodiment. Furthermore, Fig. 8 is an explanatory diagram illustrating an example of an appearance and a use form of the user terminal 50 according to the present embodiment.

As described above, the user terminal 50 can be a device such as a tablet-type PC and a smartphone. As illustrated in Fig. 7, the user terminal 50 mainly includes an input unit 500, the output unit 510, the control unit 530, and a communication unit 540. Each functional unit of the user terminal 50 will be described in detail below.

### (Input unit 500)

The input unit 500 receives an input of data and a command to the user terminal 50. More specifically, the input unit 500 is realized by a touch panel, a keyboard, and the like.

### (Output unit 510)

The output unit 510 includes, for example, a display, a speaker, a video output terminal, a voice output terminal, and the like and outputs various information by an image, voice, or the like. Note that the output unit 510 can function as the output unit 110 of the wearable device 10 as described above.

### (Control unit 530)

The control unit 530 is provided in the user terminal 50 and can control each block of the user terminal 50. The control unit 530 is realized by hardware, for example, a CPU, a ROM, a RAM, and the like.

### (Communication unit 540)

The communication unit 540 can exchange information with an external device such as the server 30. Note that the communication unit 540 is realized by a communication device such as a communication antenna, a transmission and reception circuit, and a port.

As described above, as the user terminal 50, a device such as a tablet-type PC and a smartphone can be employed. Fig. 8 illustrates an example of an appearance of a user terminal 50a which is a tablet-type PC. For example, as illustrated on the left side in Fig. 8, the user terminal 50a is attached to a treadmill 52 by using an attaching gear 54 used to attach the user terminal 50a. As illustrated in Fig. 8, by attaching the user terminal 50a, a user who works out by using the treadmill 52 can visually recognize a display as the output unit 510 of the user terminal 50a.

### <2.5. Configuration of control unit 130 according to present embodiment>

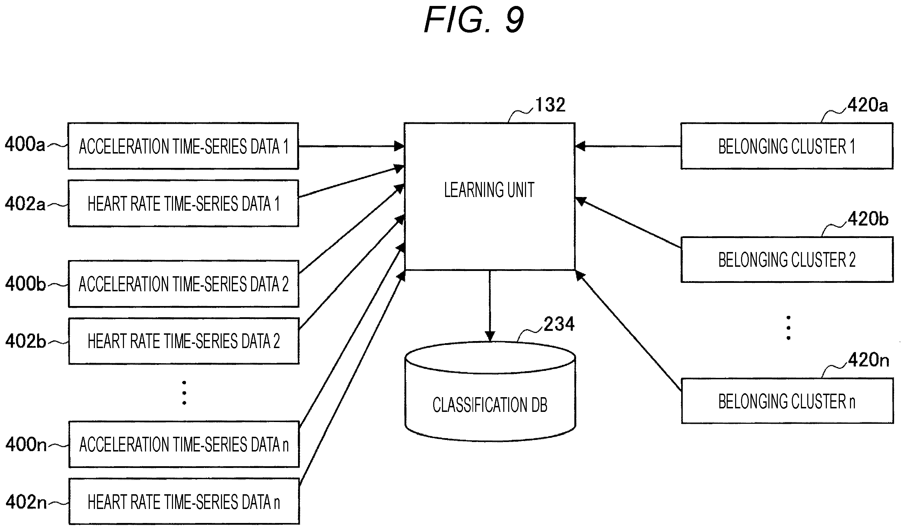

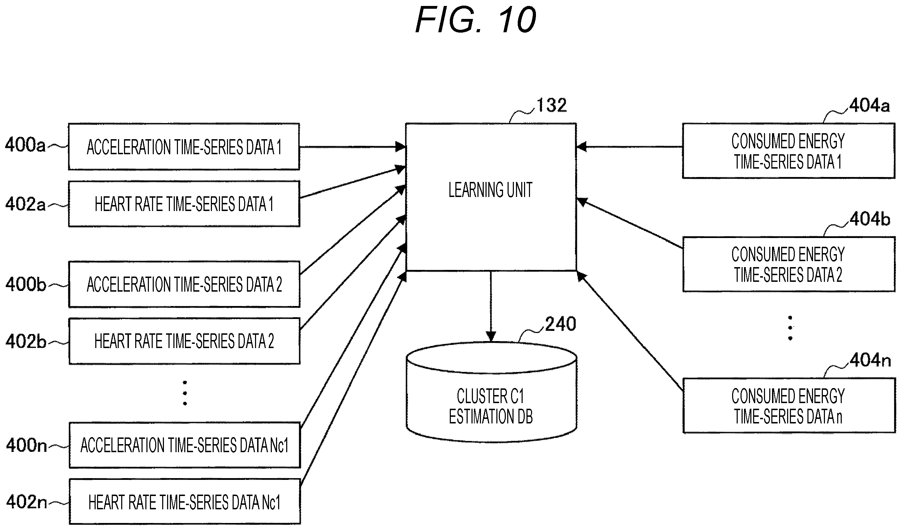

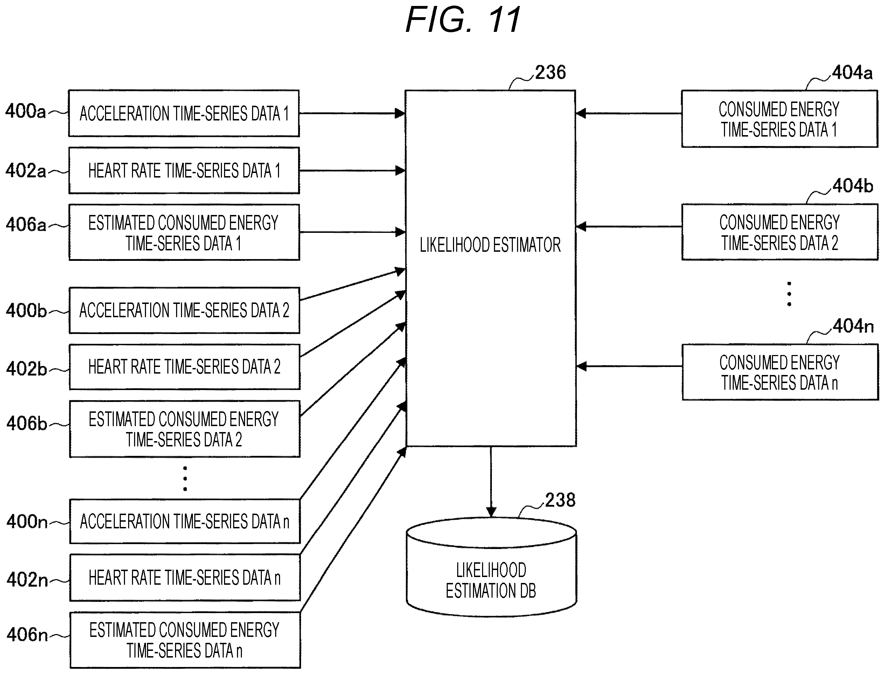

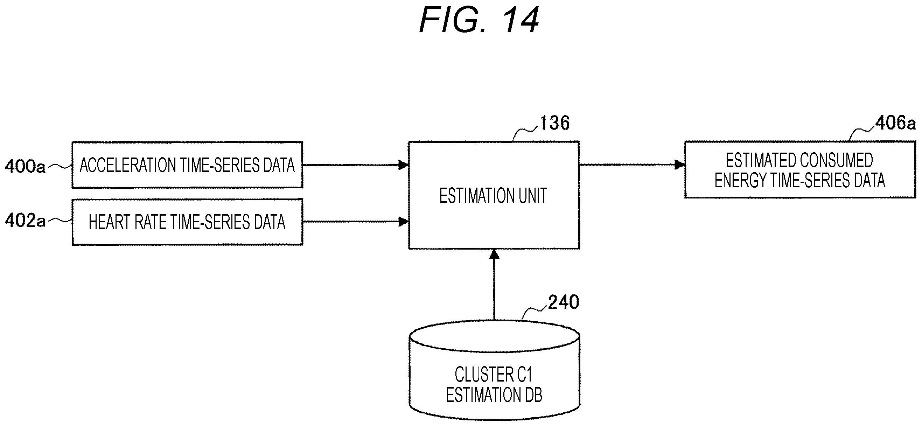

A configuration of the control unit 130 according to the present embodiment will be described below with reference to Figs. 9 to 14. Figs. 9 and 10 are explanatory diagrams for explaining an operation of the learning unit 132 according to the present embodiment. Figs. 11 to 13 are explanatory diagrams for explaining an operation of a likelihood estimator 236 according to the present embodiment. Moreover, Fig. 14 is an explanatory diagram for explaining an operation of the estimation unit 136 according to the present embodiment. As described above, the control unit 130 mainly includes three functional units, i.e., the learning unit 132, the classification unit 134, and the estimation unit 136. Each functional unit of the control unit 130 will be described below.

### (Learning unit 132)

For each cluster, the learning unit 132 performs machine learning by using the heart rate fluctuation pattern caused by a change in an exercise intensity belonging to the cluster and the consumed energy fluctuation pattern measured simultaneously with the heart rate fluctuation pattern. Then, the learning unit 132 acquires relation information indicating a relation between the heart rate fluctuation pattern caused by the change in the exercise intensity and the consumed energy fluctuation pattern by the machine learning for each cluster.

Here, the cluster indicates a group of data having similar tendency which can be estimated by using the same model. Specifically, in the present embodiment, a plurality of pieces of time-series data of the heart rate, of which the tendencies of the heart rate fluctuation pattern caused by the change in the exercise intensity are similar to each other, in other words, tendencies of patterns in which a heart rate enhancement phenomenon appears are similar to each other, is used as assuming that the plurality of pieces of time-series data belongs to the same cluster. Since the plurality of pieces of time-series data of the heart rate belonging to the same cluster has the tendencies similar to each other, it is considered that time-series data of the consumed energy can be estimated from the time-series data of the heart rate by using a common model. Therefore, the learning unit 132 acquires the relation information indicating the relation between the heart rate fluctuation pattern caused by the change in the exercise intensity and the consumed energy fluctuation pattern which is a model obtained as considering the appearance pattern of the enhancement phenomenon of the heart rate of the corresponding cluster in each cluster. Each piece of the acquired relation information is used when estimation is performed by an estimator of the estimation unit 136 as described later for each cluster. In other words, in the present embodiment, the estimator exists for each cluster and is used for the estimation by the estimator associated with the cluster. Therefore, the learning unit 132 prepares the relation information for each cluster.

More specifically, the learning unit 132 can perform learning as follows. As illustrated in Fig. 9, the learning unit 132 receives the plurality of heart rate fluctuation patterns caused by the change in the exercise intensity acquired by attaching the above-described wearable devices 10 to the plurality of users. Here, as the heart rate fluctuation pattern caused by the change in the exercise intensity, acceleration time-series data 400 indicating the change in the exercise intensity and heart rate time-series data 402 corresponding to the acceleration time-series data 400 are used. Furthermore, a cluster is applied to each of the acceleration and the heart rate time-series data 400 and 402 to be input as a label 420. Note that the data to be input to the learning unit 132 is not limited to such acceleration and the heart rate time-series data 400 and 402 and may be, for example, a heart rate fluctuation pattern caused by a known change in an exercise intensity. Moreover, the heart rate time-series data 402 to be input includes the appearance pattern of the enhancement phenomenon. Then, the learning unit 132 extracts feature points and feature amounts of the time-series data 400 and 402 in each cluster by using machine learning using a recurrent neural network and the like and generates a classification database 234. The classification database generated here can be used for searching for the cluster which is used when the consumed energy is estimated. Moreover, as illustrated in Fig. 10, to the learning unit 132, the acceleration and the heart rate time-series data 400 and 402 belonging to the same cluster and consumed energy time-series data 404 simultaneously acquired by measuring respiration (actual measurement value of consumed energy) are input. The learning unit 132 performs supervised machine learning using the recurrent neural network and the like as using the time-series data 400, 402, and 404 as input signals and teacher signals. Then, the learning unit 132 acquires the relation information indicating the relation between the acceleration and the heart rate time-series data 400 and 402 and the consumed energy time-series data 404 by the machine learning described above. The learning unit 132 constructs an estimation database 240 storing the acquired relation information for each cluster. The estimation database 240 constructed for each cluster is used for estimation of the consumed energy.

Note that the learning unit 132 may perform machine learning by a semi-supervised learning device so as to omit labeling to a part of the acceleration and the heart rate time-series data 400 and 402. In this case, the learning unit 132 can enhance a classification ability by performing learning so that the acceleration and the heart rate time-series data 400 and 402 with no labels, which are determined to be more similar to each other than the acceleration and the heart rate time-series data 400 and 402 to which the labels are applied, belongs to the same cluster. Furthermore, the learning unit 132 may inquire, for example, about the physical characteristics or the like to the user and perform soft-supervised learning by using cluster information determined on the basis of the answer to the question as a rough teacher signal. Alternatively, the learning unit 132 may perform learning with no teacher in which the cluster is automatically extracted by using a large amount of data. In this case, the learning unit 132 automatically generates the cluster.

Note that, in the present embodiment, the learning unit 132 acquires the relation information indicating the relation between the heart rate and the consumed energy by using the acceleration, the heart rate, and the consumed energy time-series data 400, 402, and 404 belonging to the same cluster. Since these time-series data 400, 402, and 404 belonging to the same cluster has the similar tendency, in other words, the similar appearance pattern of the enhancement phenomenon of the heart rate, specific relation information can be easily found from the above-described machine learning. Note that, to construct the estimation database 240 of the single cluster, it is preferable to use the time-series data 400, 402, and 404 acquired from at least several subjects. However, construction of the estimation database 240 according to the present embodiment is not limited to a method using the time-series data 400, 402, and 404 acquired by performing the measurement on the subject. For example, a part of the time-series data 400, 402, and 404 used for the construction of the database 240 may be dummy time-series data artificially reproducing the tendency in the corresponding cluster. Furthermore, in the present embodiment, the learning method by the learning unit 132 is not limited to a method using the above-described machine learning, and other learning method may be used.

### (Classification unit 134)

Before the estimation by the estimation unit 136 as described later, the classification unit 134 classifies which one of the plurality of clusters the input data belongs to. Moreover, the input data is input to the estimator for the cluster to which the data belongs and is used to estimate the consumed energy. Specifically, the classification unit 134 compares the heart rate fluctuation pattern caused by the change in the exercise intensity which has been input (for example, heart rate time-series data) with a model associated with each cluster (for example, heart rate time-series data) and searches for a model having the smallest difference between the input fluctuation pattern and the model on the basis of the comparison result. Alternatively, the classification unit 134 searches for a model which is the most similar to the fluctuation pattern. Then, the classification is performed as assuming that the heart rate fluctuation pattern belongs to the cluster for the searched model. In other words, the classification unit 134 searches for the cluster corresponding to the appearance pattern of the enhancement phenomenon having the same tendency on the basis of the appearance pattern of the enhancement phenomenon of the heart rate of each user. Since the estimator for each cluster performs estimation in consideration of the tendency of the data belonging to each cluster, in other words, the appearance pattern of the enhancement phenomenon of the heart rate, the estimation accuracy can be improved by using such an estimator.

More specifically, the classification unit 134 may use the classification database 234 generated by the learning unit 132. In this way, the classification unit 134 can search for the clusters to which the plurality of heart rate fluctuation patterns caused by the change in the exercise intensity which has been newly acquired for estimation belongs.

Furthermore, the classification unit 134 may, for example, perform the classification by estimating the likelihood. The classification by using the likelihood will be described with reference to Figs. 11 to 13. The likelihood is an index indicating reliability or certainty of the estimation according to the input data. In this case, the estimator for estimating the consumed energy is prepared for each cluster in advance. First, the acceleration time-series data 400 indicating the change in the exercise intensity and the heart rate time-series data 402 corresponding to the acceleration time-series data 400 are input to each estimator as the heart rate fluctuation patterns caused by the change in the exercise intensity, and consumed energy time-series data 406 is estimated. Note that, here, the estimated consumed energy time-series data 406 is referred to as the estimated consumed energy time-series data 406 so as to be distinguished from the consumed energy time-series data 404 acquired by measuring respiration (actual measurement value of consumed energy).

Furthermore, the classification unit 134 includes a plurality likelihood estimators 236 as illustrated in Fig. 11. The likelihood estimator 236 is provided to correspond to the estimator prepared for each cluster and calculates an estimation likelihood obtained by the corresponding estimator. Specifically, the likelihood estimator 236 receives the above-described acceleration time-series data 400, the heart rate time-series data 402, and the estimated consumed energy time-series data 406 obtained by the corresponding estimator, and the consumed energy time-series data 404 simultaneously acquired by measuring respiration. Then, the likelihood estimator 236 calculates an estimation likelihood 408 by using the input data. The likelihood estimator 236 generates a likelihood estimation database 238 including the estimation likelihood 408 acquired in this way. Each likelihood estimator 236 calculates the estimation likelihood 408 as described above.

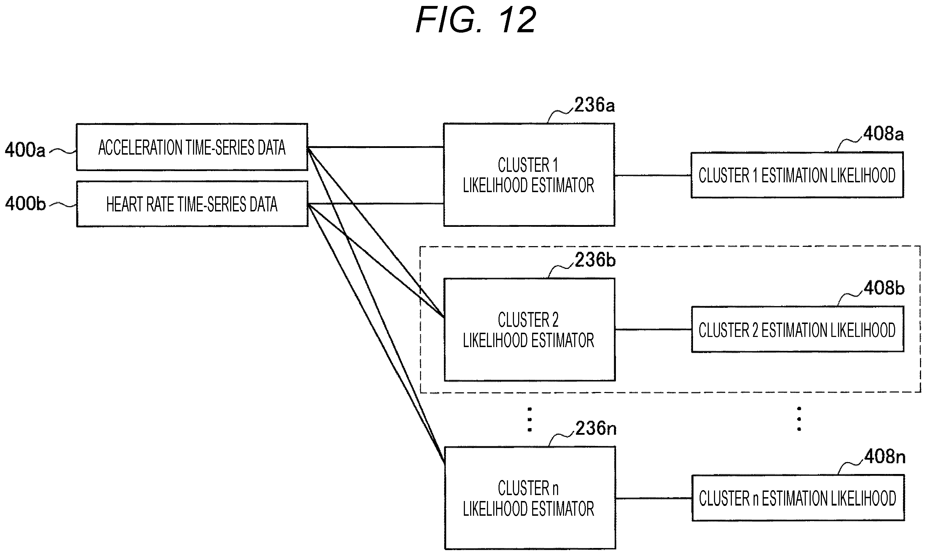

Moreover, as illustrated in Fig. 12, the classification unit 134 compares the estimation likelihoods 408 calculated by the respective likelihood estimators 236. The high estimation likelihood 408 means that the estimation is performed with high reliability by using the acceleration and the heart rate time-series data 400 and 402. Therefore, it can be said that the estimator and the cluster having the highest estimation likelihood 408 are the estimator and the cluster optimal for the acceleration and the heart rate time-series data 400 and 402. Therefore, the acceleration and the heart rate time-series data 400 and 402 is classified as data belonging to the cluster for the estimator corresponding to the likelihood estimator 236 having the highest estimation likelihood 408. For example, in the example in Fig. 12, since an estimation likelihood 408b calculated by a likelihood estimator 236b of a cluster 2 is the highest, acceleration and heart rate time-series data 400a and 402a is classified as data belonging to the cluster 2.

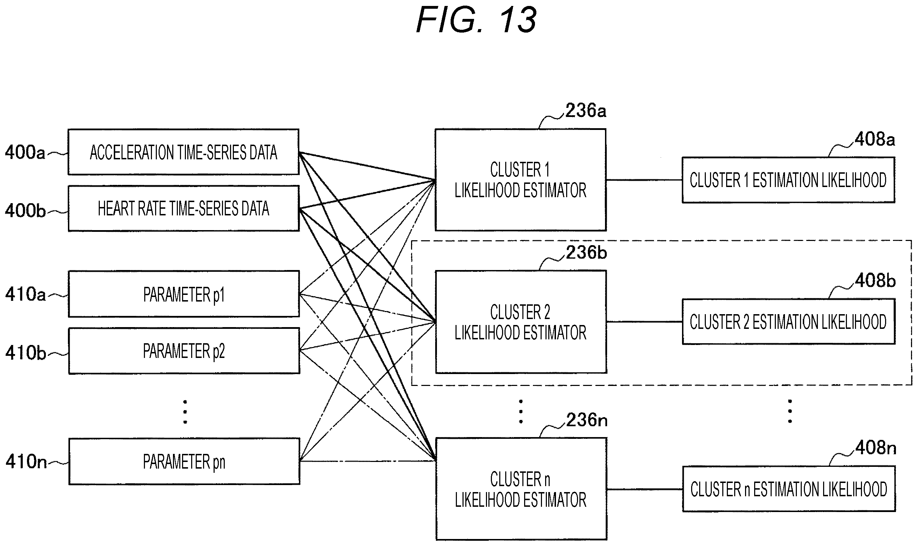

At this time, as illustrated Fig. 13, each likelihood estimator 236 may search for a numerical value of a parameter 410 which further increases the calculated estimation likelihood 408. In this case, the classification unit 134 searches for a cluster, to which the acceleration and the heart rate time-series data 400a and 402a belongs, by using the estimation likelihood 408 which is obtained after the numerical value of the parameter 410 is optimized. Furthermore, the optimized parameter 410 may be used for the estimation by the estimation unit 136 as described later. By using the optimized parameter 410 for the estimation in this way, the estimation accuracy of the consumed energy can be more improved.

Here, the parameter 410 can be, for example, a numerical value used to normalize the heart rate time-series data 400 before being input to each estimator. By normalizing the heart rate time-series data 400 to a predetermined value and inputting the value to the estimator, there is a case where the consumed energy can be estimated with higher accuracy. More specifically, as the parameter 410, a heart rate with a moderate exercise intensity can be exemplified (for example, heart rate at the time of running at about nine km/hour). It is known that the estimation accuracy is improved in a case where the heart rate time-series data 400 is normalized according to such a heart rate and the consumed energy is estimated by using the normalized heart rate time-series data 400. Therefore, for example, the likelihood estimator 236 can search for a value of the parameter 410 with which the estimation likelihood 408 is maximized by slightly changing a standard heart rate with the moderate exercise intensity (for example, average value of heart rate measured from large number of subjects) about the center value (perturbative method). Note that, instead of the search by using the perturbative method, by measuring the heart rate by making the user perform exercise with the moderate exercise intensity, a normalization parameter may be acquired.

Furthermore, as an example of the other parameter 410, a numerical value and the like used when the acceleration time-series data is normalized and a numerical value and the like used to correct the acceleration time-series data according to habit of the movement of the user and the like can be exemplified. Specifically, in the present embodiment, the acceleration is used as the index indicating the exercise intensity. However, the relation between the exercise intensity and the acceleration changes according to the position of the attachment part of the acceleration sensor, the habit of the movement by the user (for example, largely wing arms when running at low speed) and the like. Therefore, by correcting the acceleration time-series data by an appropriate parameter 410 and inputting the data to the estimator according to the attachment part of the acceleration sensor and the habit of the movement of the user, the estimation accuracy of the consumed energy can be improved. Note that, in the present embodiment, the parameter 410 searched by each likelihood estimator 236 is not limited to the above example, and may be other parameter as long as a parameter can improve the estimation accuracy of the consumed energy.

Note that, in the present embodiment, the classification method by the classification unit 134 is not limited to the above method and may be other method.

### (Estimation unit 136)

As illustrated in Fig. 14, the estimation unit 136 estimates the consumed energy time-series data 406 according to the newly acquired acceleration and the heart rate time-series data 400 and 402 of the user on the basis of the relation information stored in the estimation database 240 acquired by the learning unit 132. Since the estimation unit 136 performs estimation by using the estimation database 240 prepared for each cluster, it can be said that the estimation unit 136 includes the plurality of estimators prepared for each cluster. Specifically, the estimation unit 136 inputs the newly acquired acceleration and the heart rate time-series data 400 and 402 of the user to the estimator (estimation database 240) corresponding to the cluster to which the time-series data 400 and 402 searched by the above-described classification unit 134 belongs and performs estimation. In the present embodiment, the estimation unit 136 performs estimation on the basis of the relation information prepared for each cluster having the similar heart rate fluctuation pattern, in other words, having the similar appearance pattern of the enhancement phenomenon of the heart rate. Therefore, according to the present embodiment, since the estimation is performed in consideration of the enhancement phenomenon, the estimation accuracy of the consumed energy can be improved. Furthermore, the consumed energy estimated by the estimation unit 136 is output to the user by the output unit 110, stored in the storage unit 150, and transmitted to the server 30 and the user terminal 50.

### <2.6. Information processing method according to present embodiment>

The configuration of the information processing system 1 according to the present embodiment and the configurations of the wearable device 10, the server 30, and the user terminal 50 included in the information processing system 1 have been described in detail above. Next, an information processing method according to the present embodiment will be described. The information processing method according to the present embodiment can be divided into two stages, i.e., a learning stage in which learning is performed to acquire the relation information used for estimating the consumed energy and an estimation stage in which the consumed energy is estimated according to the physical characteristics of the user (appearance pattern of enhancement phenomenon of heart rate of user and the like).

### (Learning stage)

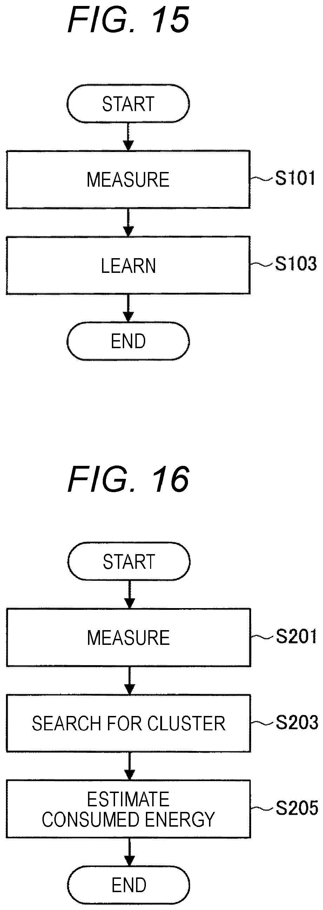

First, the learning stage will be described with reference to Fig. 15. Fig. 15 is a flow diagram for explaining an example of the learning stage in the information processing method according to the present embodiment. As illustrated in Fig. 15, the learning stage in the information processing method according to the present embodiment includes two steps, i.e., step S101 and step S103. Each step included in the learning stage of the information processing method according to the present embodiment will be described in detail below.

### (Step S101)

An instructor, the wearable device 10, or the user terminal 50 makes a plurality of users repeat to run, walk, and rest on a treadmill every several minutes and repeatedly makes the plurality of users exercise with a predetermined exercise intensity. At this time, by issuing an instruction to the user by the instructor, the wearable device 10, or the user terminal 50, it is possible to make the user perform a predetermined exercise. The predetermined exercise is an exercise through which the appearance pattern of the enhancement phenomenon of the heart rate of each user can be grasped. During the exercise, the wearable device 10 attached to a part of the body of each user measures the heart rate of each user. Moreover, the wearable device 10 measures the acceleration due to the exercise of the user by using the built-in acceleration sensor. Furthermore, a breath analyzer attached to a face of each user measures an oxygen concentration and a carbon dioxide concentration included in the breath of each user. With such measurement, the time-series data including the appearance pattern of the enhancement phenomenon of the heart rate used for learning by the learning unit 132 can be acquired.

### (Step S103)

The wearable device 10 performs machine learning for each cluster by using the acceleration and the heart rate time-series data 400 and 402 belonging to the cluster and the consumed energy time-series data 404 simultaneously measured in step S101. The wearable device 10 acquires the relation information indicating the relation between the acceleration and the heart rate time-series data 400 and 402 and the consumed energy time-series data 404 by the machine learning. Moreover, the wearable device 10 constructs the estimation database 240 storing the acquired relation information for each cluster. Note that, since the learning in step S103 has been described in detail above, the description is omitted here.

Note that, in the exercise in step S101, it is not necessary to use a dedicated exercise device such as a treadmill, and for example, the exercise may be jogging at a constant speed and the like.

### (Estimation stage)

Next, the estimation stage will be described with reference to Fig. 16. Fig. 16 is a flow diagram for explaining an example of the estimation stage in the information processing method according to the present embodiment. As illustrated in Fig. 16, the estimation stage in the information processing method according to the present embodiment mainly includes a plurality of steps from step S201 to step S205. Each step included in the estimation stage of the information processing method according to the present embodiment will be described in detail below.

### (Step S201)

The wearable device 10 attached to a part of the body of the user measures the acceleration and the heart rate.

### (Step S203)

The wearable device 10 searches for a cluster to which the acceleration time-series data 400 and the heart rate time-series data 402 of the user acquired in step S201 belong. Note that, since the searching method has been described above, the description is omitted here. Furthermore, the wearable device 10 refers to past history information of the user and may use a cluster classified in the past as the cluster to which the acceleration time-series data 400 and the heart rate time-series data 402 of the user acquired in step S201 belong. Furthermore, a cluster input from the user by the input unit 100 may be the cluster to which the user belongs. In this way, the search for the cluster can be omitted. Moreover, it is possible to present an estimated value of the consumed energy to the user in a short time.

### (Step S205)

The wearable device 10 estimates the consumed energy by the estimator regarding the cluster selected in step S203 by using the acceleration and the heart rate time-series data 400 and 402 acquired in step S201.

Note that, in the example described above, the acceleration measured by the acceleration sensor is used as an index regarding the exercise intensity. However, the present embodiment is not limited to this, and for example, a user may input an index indicating the exercise intensity instead of the acceleration. Furthermore, the two divided stages including the learning stage and the estimation stage have been described above. However, these stages may be continuously performed or alternately and repeatedly performed.

As described above, according to the present embodiment, the consumed energy can be estimated with high accuracy. Specifically, in the present embodiment, the user is classified into clusters according to the tendency of the fluctuation in the relation between the consumed energy and the heart rate, in other words, the appearance pattern of the enhancement phenomenon of the heart rate. Since the tendencies of the fluctuations of the relation between the consumed energy and the heart rate of the plurality of users belonging to the same cluster are similar to each other, it is possible to grasp the tendency of the other user belonging to the same cluster by referring to a tendency of a fluctuation in a relation of a certain user in the cluster. Therefore, by using the tendency of the fluctuation in the relation of the certain user, the consumed energy of the other user in the same cluster can be estimated. Moreover, since the tendency of the fluctuation in the relation between the consumed energy and the heart rate according to the user, in other words, the appearance pattern of the enhancement phenomenon of the heart rate is considered in the estimation, the estimation accuracy of the consumed energy can be improved. Note that, by acquiring the tendency of the fluctuation in the relation of the certain user to be referred by measuring respiration in advance, the estimation accuracy of the consumed energy of the other user in the same cluster can be improved. In other words, in the present embodiment, regarding the other user, the consumed energy can be estimated without measuring respiration.

In particular, consumed energy in individual exercise for a short time and consumed energy in exercise (movement) in daily life with a low exercise intensity, which have been difficult to estimate by a device which has been examined by the present inventors so far, can be estimated with high accuracy according to the present embodiment.

Note that it is not necessary for the wearable device 10 to construct the estimation database 240 in the above-described learning stage, and the database 240 may be constructed by the server 30 and the like and stored in the storage unit 150 at the time of, for example, manufacturing and shipping the wearable device 10.

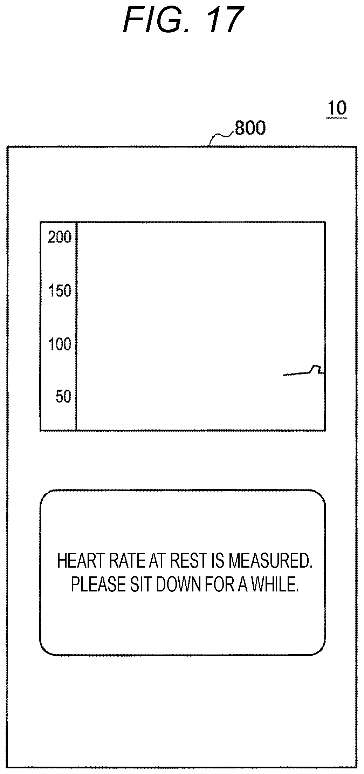

Note that the measurement in step S101 or step S201 can be performed by the instruction to the user to perform the desired exercise by the instructor and the like. However, an exercise application provided by the wearable device 10 and the like according to the present embodiment may be used. Specifically, it is possible to perform by explicitly instructing the user to perform the exercise at a predetermined exercise intensity by the exercise application. In this case, since the exercise intensity of the exercise performed by the user is known, the acceleration measurement by the acceleration sensor incorporated in the wearable device 10 can be omitted. Such an exercise application will be described below with reference to Figs. 17 to 20. Figs. 17 to 20 are explanatory diagrams for explaining examples of display screens 800 to 806 used when the time-series data is acquired.

Specifically, the exercise application provided by the wearable device 10 or the user terminal 50 according to the present embodiment displays the screen 800 which prompts the user to maintain a resting state so as to measure the heart rate of the user at rest. For example, as illustrated in Fig. 17, the screen 800 includes a message such as "Heart rate at rest is measured. Please sit down for a while" so as to prompt the user to maintain the resting state. Furthermore, in an upper part in the screen 800, a temporal change in the measured heart rate and the like are displayed.

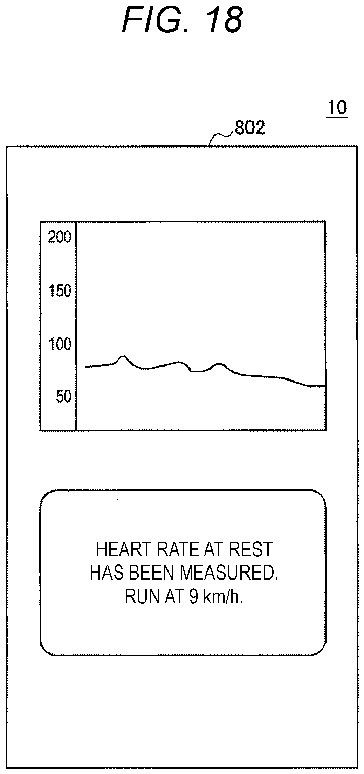

Next, in a case where the measurement of the heart rate of the user at rest has been completed, the exercise application displays the screen 802 for prompting the user to perform the exercise with the predetermined exercise intensity. For example, as illustrated in Fig. 18, the screen 802 includes a message such as "Heart rate at rest has been measured. Please run at nine km/h" so as to prompt the user to perform the exercise with the predetermined exercise intensity. Then, the user prompted by such a screen 802 performs a designated exercise by using the treadmill 52 and the like.

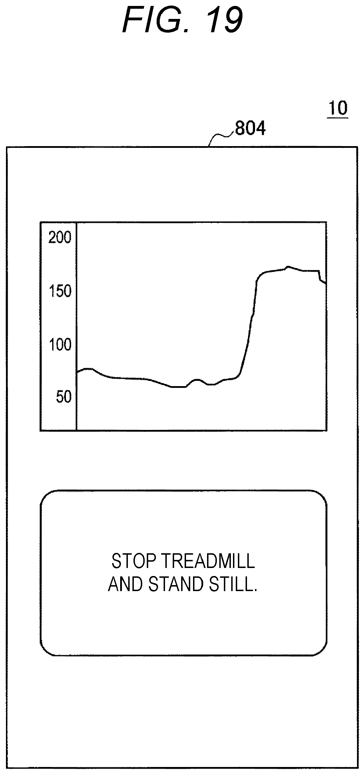

Moreover, after the heart rate in the exercise with the predetermined exercise intensity is measured, the exercise application displays the screen 804 for prompting the user to terminate the exercise and maintain the resting state so as to acquire the appearance pattern of the enhancement phenomenon of the heart rate after the exercise is terminated. As illustrated in Fig. 19, the screen 804 includes a message such as "Stop treadmill and stand still" so as to prompt the user to terminate the exercise and maintain the resting state.

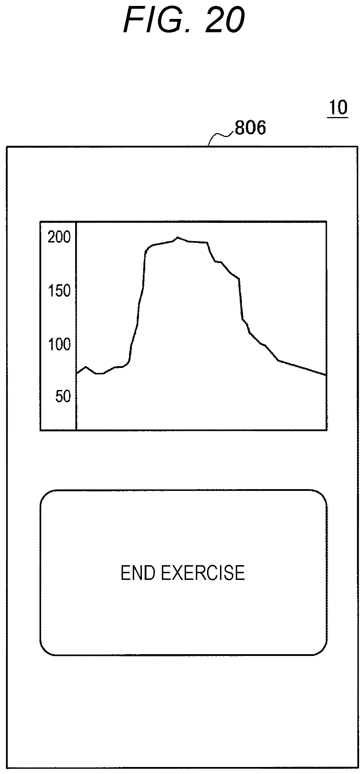

Next, after the appearance pattern of the enhancement phenomenon of the heart rate is acquired, the exercise application displays the screen 806 for notifying the user that the measurement is terminated. As illustrated in Fig. 20, the screen 806 includes a message such as "End exercise" so as to notify the user that the measurement is terminated. Note that the screens 800 to 806 illustrated in Figs. 17 to 20 described above are merely examples, and the present embodiment is not limited to such screens.

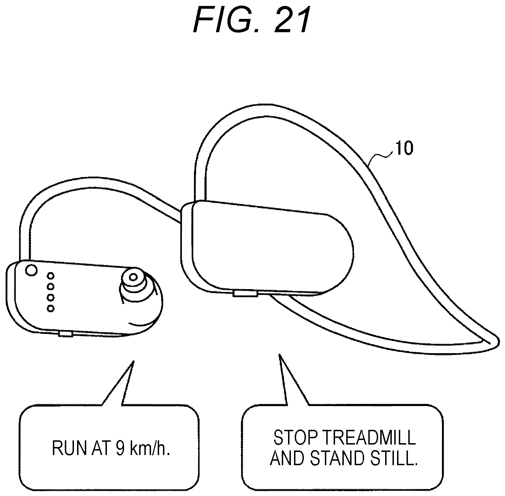

In this way, the exercise application can make the user perform the exercise in step S201. Note that, in the above description, it is assumed that the wearable device 10 and the like have a display. However, even in a case where the wearable device 10 does not have the display, it is possible to make the user perform the exercise in step S201. For example, as illustrated in Fig. 21 which is an explanatory diagram for explaining a method for guiding the user to perform the exercise when the time-series data is acquired, the wearable device 10 may output voice information to the user according to the exercise application.

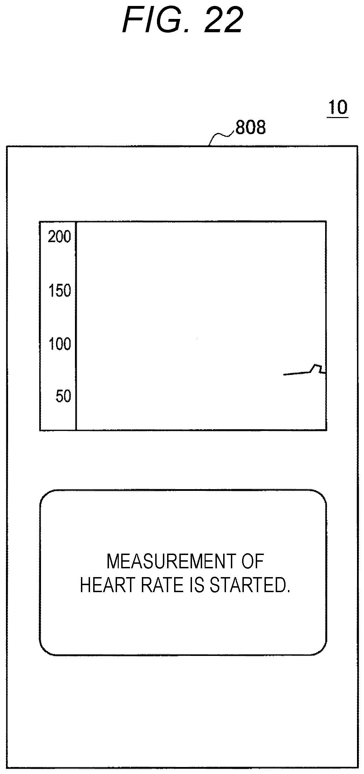

Furthermore, the measurement in step S101 or step S201 is not limited to the measurement performed by the explicit instruction by the exercise application described above. For example, the time-series data including the appearance pattern of the enhancement phenomenon of the heart rate may be acquired by detecting a change point and a stable section of the exercise intensity caused by the movement of the user by using the acceleration sensor of the wearable device 10 instead of the explicit instruction. In this case, the acceleration sensor detects the stable section in which the exercise intensity is constant and acquires the heart rate measured at this time as the time-series data. Such a method will be described below with reference to Figs. 22 to 25. Figs. 22 to 25 are explanatory diagrams for explaining examples of display screens 808 to 814 used in other method for acquiring the time-series data.

Specifically, in a case where the wearable device 10 detects that the user is in the resting state for a predetermined period of time (for example, several minutes) or longer, the wearable device 10 starts to measure the heart rate of the user. At this time, as illustrated in Fig. 22, the wearable device 10 displays the screen 808 for notifying the user that the measurement is started, for example, a message indicating "Measurement of heart rate is started".

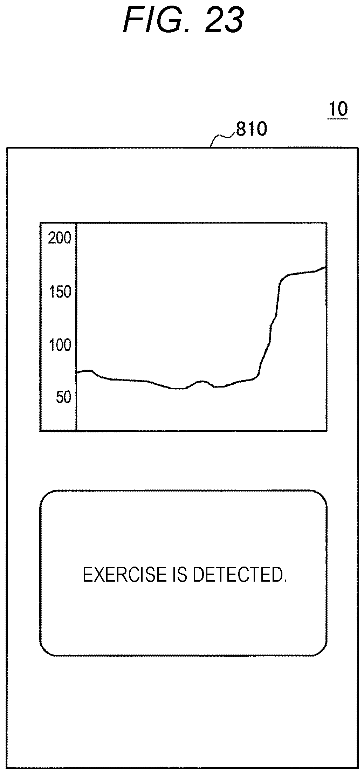

Moreover, in a case where the exercise with an exercise intensity equal to or higher than the predetermined exercise intensity by the user is detected, the wearable device 10 displays the screen 810 including a message such as "Exercise is detected" as illustrated in Fig. 23 so as to notify that the measurement is started.

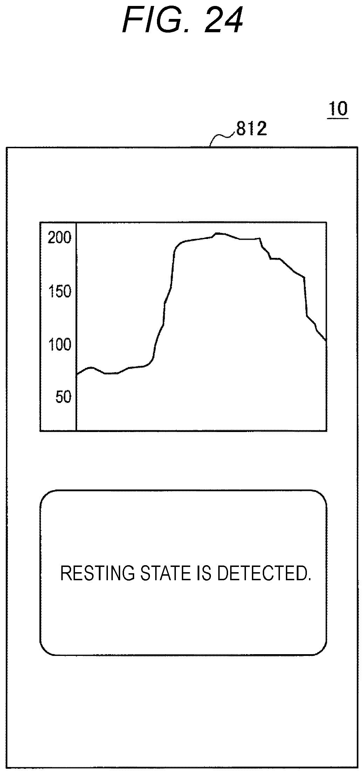

Next, in a case where the wearable device 10 detects that the user terminates the exercise and is in the resting state, the wearable device 10 displays the screen 812 including a message such as "Resting state is detected" as illustrated in Fig. 24 and measures the enhancement phenomenon of the heart rate of the user after the exercise is terminated.

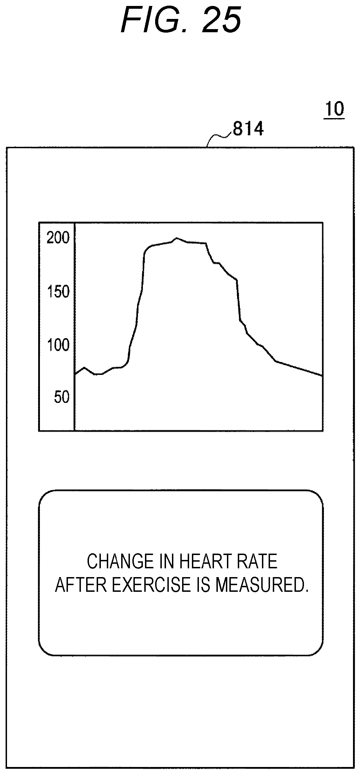

Moreover, in a case where the appearance pattern of the enhancement phenomenon of the heart rate of the user is detected in the series of measurements described above, the wearable device 10 displays the screen 814 such as "Change in heart rate after exercise is measured" as illustrated in Fig. 25. Note that the screens 808 to 814 illustrated in Figs. 22 to 25 described above are merely examples, and the present embodiment is not limited to such screens.

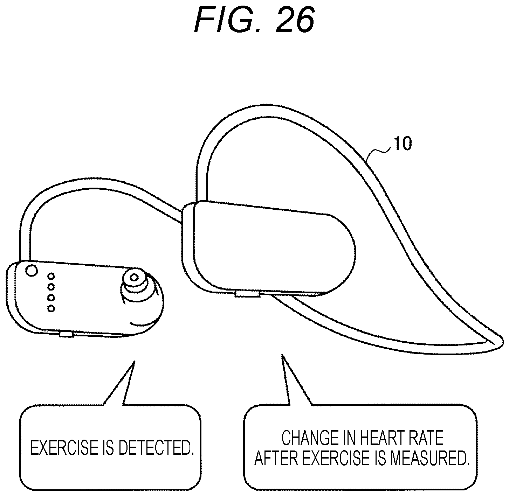

In this way, by using the acceleration sensor, the time-series data including the appearance pattern of the enhancement phenomenon of the heart rate can be acquired according to daily activities and daily exercise of the user without instructing the user to perform the exercise with the predetermined exercise intensity. Note that, in the above description, it is assumed that the wearable device 10 and the like includes the display. However, the present embodiment is not limited to the wearable device 10 and the like having the display. For example, as illustrated in Fig. 26 which is an explanatory diagram for explaining other method for guiding the user to perform the exercise when the time-series data is acquired, the wearable device 10 may output voice information to the user according to the exercise application.

Note that, in the exercise in step S201, it is not necessary to use a dedicated exercise device such as a treadmill, and for example, the exercise may be jogging at a constant speed and the like.

### <<3. Examples according to present embodiment>>

The information processing method according to the embodiment of the present disclosure has been described above in detail. Moreover, a specific example of an application for providing useful information to the user by using the above-described present embodiment will be described. Note that examples to be indicated below are merely examples of the application, and information processing according to the present embodiment is not limited to the following examples.

### <3.1. Example 1>

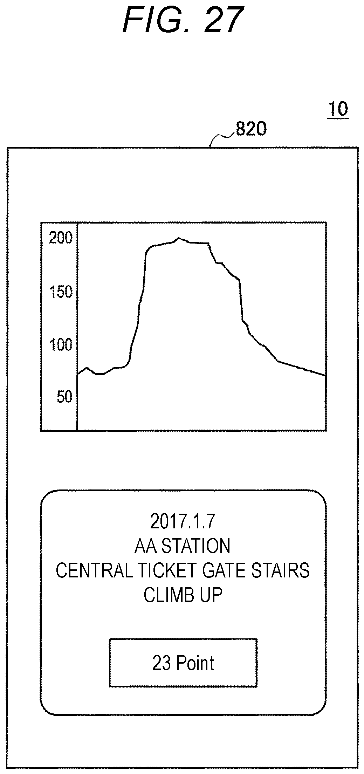

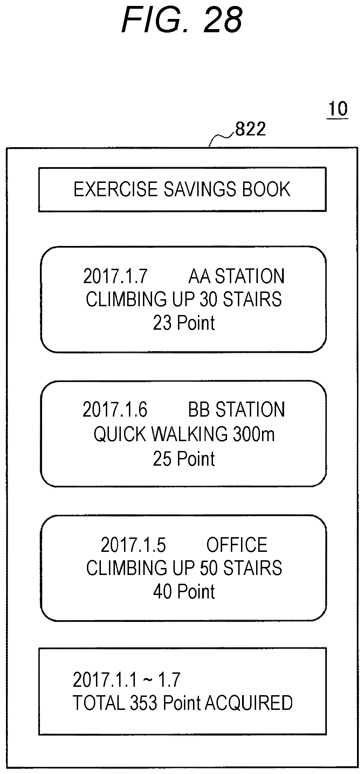

The examples to be described below relate to an application for providing useful information to the user since the estimation accuracy of the consumed energy for each exercise is improved by the above-described present embodiment. Such an example 1 will be described with reference to Figs. 27 and 28. Figs. 27 and 28 are explanatory diagrams for explaining examples of display screens 820 and 822 of the example 1 according to the present embodiment.

In the present embodiment, as described above, the estimation accuracy of the consumed energy of the individual exercise in a short time is improved. Therefore, for example, it is possible to grasp a difference between consumed energy at the time of walking on a slope and consumed energy at the time of walking on a flat road and the like. Therefore, it is possible to estimate the consumed energy in daily activity of the user which is a series of short-time exercises (micro exercise) with high accuracy by using the estimation according to the above-described present embodiment and provide useful information to the user on the basis of the estimated consumed energy. Such examples will be described below.

### (Example 1A)

In the present example, the estimated consumed energy for each micro exercise is notified to the user. For example, in a case where a user's exercise (activity), which continues for a certain period of time (equal to or longer than several minutes), of which consumed energy equal to or more than a predetermined threshold is estimated, is detected, the wearable device 10 notifies the user of the estimated consumed energy caused by the exercise. At this time, the wearable device 10 can make a notification to the user, for example, by displaying the screen 820 illustrated in Fig. 27. Furthermore, the wearable device 10 can acquire positional information where the user has performed the exercise by using a GPS receiver and the like built in the wearable device 10. Moreover, since the wearable device 10 includes a gyro sensor, a geomagnetic sensor, and the like in addition to the acceleration sensor, it is possible to acquire detailed information of content of the exercise performed by the user by analyzing sensing information acquired from these sensors. Therefore, the wearable device 10 can notify the user of not only the estimated consumed energy but also the positional information where the user has performed the exercise and the detailed information of the content of the exercise.

More specifically, in Fig. 27, the screen 820 displaying the consumed energy at the time when the user uses stairs at a central ticket gate of an AA station is illustrated. Note that, here, it is assumed that the stairs are provided together with an escalator. Therefore, the wearable device 10 refers to the past exercise history and the estimated consumed energy of the user and acquires the estimated consumed energy consumed at the time when the user uses the escalator on another day. Then, the wearable device 10 calculates a difference between the estimated consumed energy at the time when the user uses the stairs and the estimated consumed energy at the time when the user uses the escalator and notifies the user of the calculated difference as points. For example, the notified point is displayed at the bottom of the screen 820 in Fig. 27. The point calculated as described above indicates how much the energy can be consumed in a case where the user performs the exercise (activity) applying a higher load to the user's body than the energy consumed in a case where the exercise which applies no load to the body is selected in a form which can be easily understand by the user. The notification of such a point to the user makes the user feel small sense of accomplishment by performing the exercise applying the load to the body and enhances motivation of the user to select the exercise applying the load to the body.

### (Example 1B)

Next, an example in which the above-described point is notified to the user as an "exercise savings book" will be described with reference to Fig. 28. In Fig. 28, the screen 822 is illustrated which displays a list of the estimated consumed energy for each exercise performed by the user and the point corresponding to the estimated energy for the user as the "exercise savings book". Specifically, in the upper part of the screen 822, as in Fig. 27, the consumed energy at the time when the user uses the stairs in the AA station and the point on the basis of the consumed energy are illustrated. Furthermore, in the middle part of the screen 822, consumed energy consumed by quick walking (more specifically, walking at speed faster than standard walking speed) in a BB town by the user and the point on the basis of the consumed energy are illustrated. The point in this case corresponds to a difference, for example, from estimated consumed energy when the user walks at a standard walking speed for a distance equal to the distance of the quick walking above. Moreover, in the bottom of the screen 822, a total sum of the points obtained by the user in a predetermined period (for example, one day, one week, and the like) is illustrated. In other words, in the present example, the estimated consumed energy caused by the micro exercise which is individually detected and the point acquired by the micro exercise are notified to the user as the savings book as illustrated in Fig. 28. By notifying the point in this way, the user thinks to acquire more points. Therefore, it is possible to lead the user to naturally perform the exercise applying the load to the user's body (micro exercise).

Moreover, by using the estimation of the consumed energy according to the present embodiment, it is possible to recommend the exercise applying the load to the body (micro exercise) to the user. For example, in the present example, in a case of detecting that the user has reached the position where the estimated consumed energy equal to or more than the predetermined threshold is acquired by the exercise of the user in the past, the wearable device 10 recommends the user to perform the exercise related to the estimated consumed energy equal to or more than the predetermined threshold.

More specifically, in a case of detecting that the user has reached the position (for example, stairs) where the estimated consumed energy equal to or more than the predetermined threshold is acquired by the exercise of the user in the past by the built-in GPS receiver, the wearable device 10 refers to the total sum of the estimated consumed energy of the user on that day. In a case where the total sum of the estimated consumed energy of the user on that day is less than a target value of consumed energy of the user of a day as a result of referring to the total sum of the estimated consumed energy, the wearable device 10 recommends the user to perform the exercise (for example, "climbing up stairs") related to the estimated consumed energy equal to or more than the predetermined threshold. More specifically, in a case where the wearable device 10 is a bracelet type device, vibration of a vibration device provided as the output unit 310 of the wearable device 10 may recommend the user to perform the exercise, that is, the "climbing up stairs". Furthermore, in a case where the wearable device 10 includes a display as the output unit 310, it is possible to recommend the user to perform the "climbing up stairs" by displaying a display such as "Recommendation" at the position of the stairs on a map displayed on the display.

Note that the wearable device 10 may recommend the micro exercise, for example, on the basis of the target value of the consumed energy of the user on one day or the number of acquired points. Furthermore, the wearable device 10 may determine whether or not to make a recommendation on the basis of the record of the micro exercise of the user in the past. For example, in a case where the contents of the micro exercise actually performed by the user according to the recommendation has a specific tendency as referring to the history in the past, the wearable device 10 recommends the micro exercise according to the tendency. Furthermore, the wearable device 10 may detect the position where the estimated consumed energy equal to or more than the predetermined threshold is acquired by exercise of other user by referring to information of the other user accumulated in the server 30.

As described above, according to the estimation according to the present embodiment, the consumed energy of the exercise (movement) in the daily life with a low exercise intensity can be estimated with high accuracy. Therefore, by using the estimation, in the present example, it is possible to recommend the movement (for example, "climbing up stairs" and the like) normally performed in daily life, not a special exercise (for example, running and the like) with a high exercise intensity, as the micro exercise.

### <3.2. Example 2>

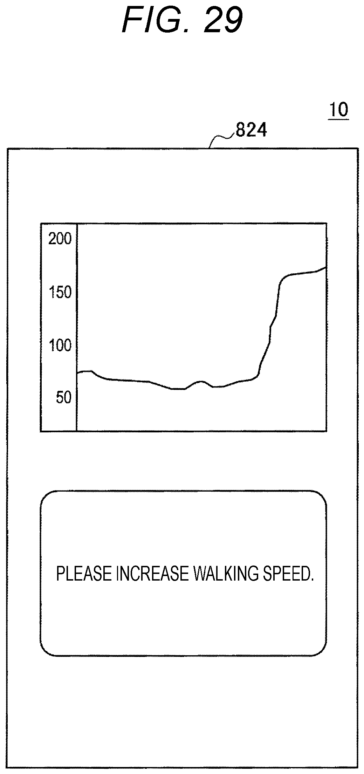

According to the estimation of the present embodiment, it is possible to determine whether or not the enhancement phenomenon of the heart rate appears. Furthermore, by using the estimation according to the present embodiment described above, the user can estimate conditions (exercise intensity and the like) and the like in which the enhancement phenomenon of the heart rate appears. Therefore, by using the above determination and estimation, it is possible to create an application which notifies the user of the appearance of the enhancement phenomenon and recommends the exercise for making the enhancement phenomenon appear. The examples described below relate to such an application. Such an example 2 will be described with reference to Figs. 29 to 32. Figs. 29 to 32 are explanatory diagrams for explaining examples of display screens 824 to 830 of the example 2 according to the present embodiment.

### (Example 2A)

For example, the wearable device 10 can estimate consumed energy in consideration of the enhancement state from the newly acquired acceleration and heart rate time-series data 400 and 402 of the user by the estimation of the present embodiment. On the other hand, by applying the consumed energy estimation according to the related art which has been studied by the present inventors so far to the newly acquired acceleration and the heart rate time-series data 400 and 402 of the user, it is possible to estimate the consumed energy without considering the enhancement phenomenon. Therefore, for example, by detecting that a difference between an estimated value in consideration of the enhancement state and an estimated value without considering the enhancement state exceeds a certain threshold, the wearable device 10 can notify the user that the enhancement phenomenon appears.

Therefore, in the present example, in a case where the termination of the exercise of the user is detected and the heart rate is lowered after the detection, the wearable device 10 determines whether or not the enhancement phenomenon of the heart rate appears and notifies the user of the determination result. Since the enhancement phenomenon of the heart rate appears by applying the high load to the user's body, the user can feel a sense of accomplishment such that the user can perform the exercise for making the enhancement phenomenon by notifying that the enhancement phenomenon of the heart rate appears. Moreover, by making such a notification, it is possible to increase the motivation for the user to perform the exercise which applies the load to the body.

### (Example 2B)

Furthermore, as described above, the wearable device 10 can estimate the conditions (exercise intensity and the like) and the like in which the enhancement phenomenon of the heart rate of the user appears by using the estimation according to the above-described present embodiment. Specifically, by referring to the record of the fluctuation pattern of the exercise intensity of the user in the past and the fluctuation pattern of the heart rate at that time, the wearable device 10 can estimate the degree of exercise intensity of the exercise which makes the enhancement phenomenon of the heart rate appear (condition). Therefore, the wearable device 10 can recommend the exercise which makes the enhancement phenomenon of the heart rate appear to the user on the basis of the exercise intensity with which the enhancement phenomenon of the estimated heart rate appears.

In the present example, the wearable device 10 detects that the user is walking and compares with the above-described estimated conditions. In a case where it is estimated that the enhancement phenomenon of the heart rate does not appear by the detected walking, the wearable device 10 recommends the exercise which makes the enhancement phenomenon of the heart rate appear to the user. For example, as illustrated in Fig. 29, the wearable device 10 displays the screen 824 including a message such as "Please increase walking speed" and "Keep current speed for two minutes", and leads the user to perform the exercise for making the enhancement phenomenon of the heart rate appear. Furthermore, in the present example, the recommendation of the exercise is not limited to a recommendation made by the display of the screen as described above, and the exercise may be recommended to the user by voice information, vibration, and the like. Such recommendation increases an opportunity for the user to perform the exercise having the exercise intensity for making the enhancement phenomenon of the heart rate appear.

### (Example 2C)

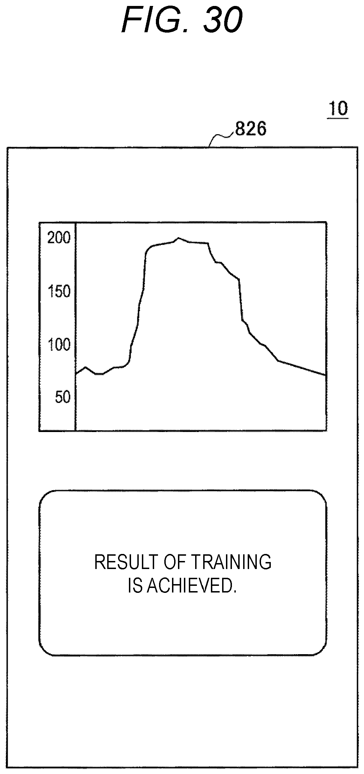

Furthermore, in the next example, the wearable device 10 detects that the user performs exercise and compares the exercise with the above-described estimated conditions (exercise intensity with which enhancement phenomenon of heart rate appears). In a case where it is estimated that the detected exercise makes the enhancement phenomenon of the heart rate appear, the wearable device 10 determines whether or not the enhancement phenomenon of the heart rate appears. Then, in a case where it is determined that the enhancement phenomenon of the heart rate does not appear, the wearable device 10 makes the notification to the user. More specifically, as illustrated in Fig. 30, the wearable device 10 displays the screen 826 including a message such as "Result of training is achieved" and "Heart rate is more smoothly returned than before", so as to make the notification to the user.

In a case where the enhancement phenomenon of the heart rate does not appear when the exercise with the exercise intensity which has caused the enhancement phenomenon of the heart rate in the past appears is newly performed by the user, it is surmised that the user's physical ability (respiratory function and the like) is improved. On the basis of such surmise, in the present example, the user can feel the improvement of the user's physical ability by the notification described above.

### (Example 2D)

Furthermore, as described above, in a case where the user's physical ability is improved, the cluster belonging to the fluctuation pattern of the heart rate caused by the change in the exercise intensity obtained from the user changes, and the estimator used to estimate the consumed energy according to the fluctuation pattern is switched in response to the change of the cluster. Therefore, an example in which the user can feel the improvement in the user's physical ability by making the notification in a case where the cluster changes in this way will be described below.

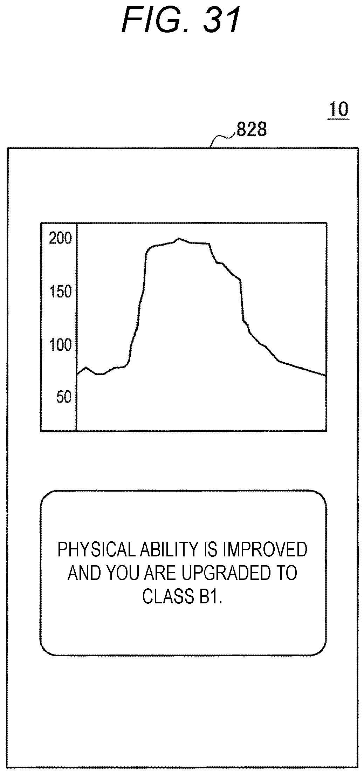

More specifically, the wearable device 10 extracts the plurality of recent pieces of acceleration and heart rate time-series data 400 and 402 of the user. Moreover, the wearable device 10 estimates the cluster likelihood illustrated in Fig. 12 by using the extracted time-series data 400 and 402. Then, in a case where the specified cluster having the highest likelihood can be considered as a cluster having a higher physical ability than the clusters in the past, the wearable device 10 makes a notification to the user. More specifically, the wearable device 10 displays the screen 828 including a message such as "Physical ability is improved and you are upgraded to class B1" as illustrated in Fig. 31, and the cluster is switched. Then, it is notified that the physical ability is estimated to be improved from the result. In the present example, the user can feel the improvement of the user's physical ability by the notification described above.

Note that, in the above description, the example focusing on the cluster has been described. However, the present example is not limited to this and may focus on the parameter 410 which is optimized at the time of classification. In this case, in a case where the numerical value of the parameter 410 which has been newly optimized is changed from the numerical value of the parameter 410 used for estimation in the past, it can be surmised that the user's physical ability is changed.

### (Example 2E)

In the example 2C described above, whether or not the enhancement phenomenon appears in the exercise with the predetermined exercise intensity is changed according to the user's physical ability. However, whether or not the enhancement phenomenon appears is changed according to a physical condition of the user. Therefore, in the present example, information regarding the physical condition of the user can be notified to the user on the basis of such an idea.

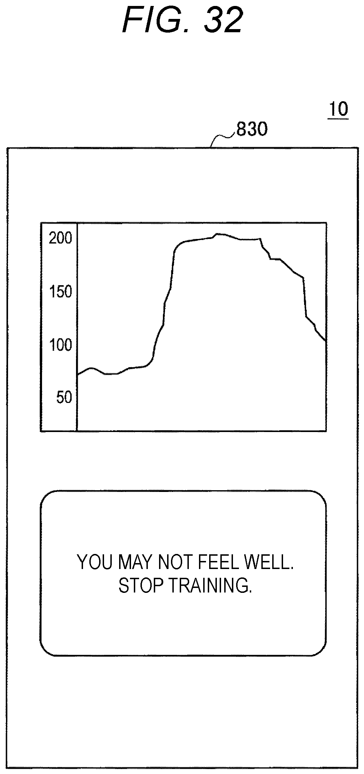

Specifically, by referring to the record of the fluctuation pattern of the exercise intensity of the user in the past and the fluctuation pattern of the heart rate at that time, the wearable device 10 can estimate the degree of the exercise intensity of the exercise which does not make the enhancement phenomenon of the heart rate appear (condition). Therefore, the wearable device 10 detects that the user performs an exercise and compares the exercise with the above-described estimated conditions (exercise intensity with which enhancement phenomenon of heart rate does not appear). In a case where it is estimated that the detected exercise makes the enhancement phenomenon of the heart rate appear, the wearable device 10 determines whether or not the enhancement phenomenon of the heart rate appears. Then, in a case where it is determined that the enhancement phenomenon of the heart rate appears, the wearable device 10 estimates that the enhancement phenomenon appears due to the bad physical condition of the user even under the condition in which the enhancement phenomenon of the heart rate does not normally appears and makes a notification to the user. More specifically, as illustrated in Fig. 32, the wearable device 10 displays the screen 830 including a message such as "You may not feel well. Stop training" and "Today, the heart rate does not recover well after exercise". By making such a notification, the user can grasp a bad physical condition which has not been recognized by oneself and can avoid unreasonable training.

### <<4. Summary>>

As described above, according to the present embodiment, the consumed energy can be estimated with high accuracy. Specifically, in the present embodiment, since the estimation is performed in consideration of the tendency of the fluctuation in the relation between the consumed energy and the heart rate according to the user, in other words, the appearance pattern of the enhancement phenomenon of the heart rate, the estimation accuracy of the consumed energy can be improved.

In the embodiment, the consumed energy may be estimated by using learning by deep neural network (DNN) and the like using a large amount of data. Even in this case, since the estimation is made in consideration of the appearance pattern of the enhancement phenomenon of the heart rate according to the user, the estimation accuracy of the consumed energy can be improved.

### <<5. Regarding hardware configuration>>

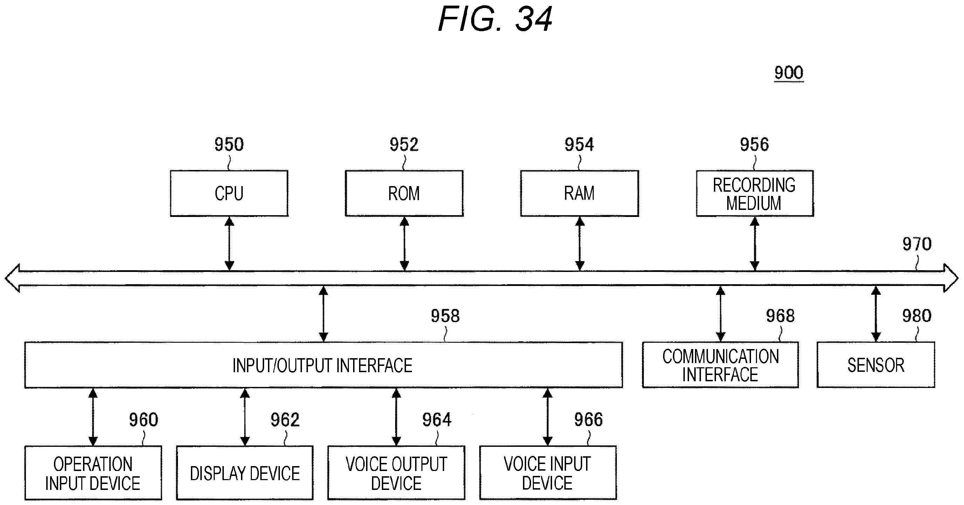

Fig. 34 is an explanatory diagram illustrating an exemplary hardware configuration of an information processing apparatus 900 according to the present embodiment. In Fig. 34, the information processing apparatus 900 indicates an exemplary hardware configuration of the above-described wearable device 10.

The information processing apparatus 900 includes, for example, a CPU 950, a ROM 952, a RAM 954, a recording medium 956, an input/output interface 958, and an operation input device 960. Moreover, the information processing apparatus 900 includes a display device 962, a voice output device 964, a voice input device 966, a communication interface 968, and a sensor 980. Furthermore, the information processing apparatus 900 connects between the components, for example, by a bus 970 as a data transmission path.

### (CPU950)

The CPU 950 is configured of one or two or more processors, various processing circuits, or the like including an arithmetic circuit, for example, a CPU, and functions as a control unit (for example, control unit 130 described above) which controls the entire information processing apparatus 900. Specifically, in the information processing apparatus 900, the CPU 950 functions, for example, as the learning unit 132, the classification unit 134, the estimation unit 136, and the like described above.

### (ROM 952 and RAM 954)

The ROM 952 stores control data and the like such as a program and a calculation parameter to be used by the CPU 950. The RAM 954 temporarily stores, for example, the program and the like executed by the CPU 950.

### (Recording medium 956)

The recording medium 956 functions as the storage unit 150 described above and stores, for example, various data such as data regarding the information processing method according to the present embodiment and various applications, and the like. Here, as the recording medium 956, for example, a magnetic recording medium such as a hard disk, and a nonvolatile memory such as a flash memory can be exemplified. Furthermore, the recording medium 956 may be detachable from the information processing apparatus 900.

### (Input/output interface 958, operation input device 960, display device 962, voice output device 964, and voice input device 966)

The input/output interface 958 connects, for example, the operation input device 960, the display device 962, and the like. The input/output interface 958 is, for example, a universal serial bus (USB) terminal, a digital visual interface (DVI) terminal, a highdefinition multimedia interface (HDMI) (registered trademark) terminal, various processing circuits, and the like.

The operation input device 960 functions, for example, as the input unit 100 described above and is connected to the input/output interface 958 in the information processing apparatus 900.

The display device 962 functions, for example, as the output unit 110 described above, is provided on the information processing apparatus 900, and is connected to the input/output interface 958 in the information processing apparatus 900. The display device 962 is, for example, a liquid crystal display, an organic electro-luminescence (EL) Display, and the like.

The voice output device 964 functions, for example, as the output unit 110 described above, is provided, for example, on the information processing apparatus 900, and is connected to the input/output interface 958 in the information processing apparatus 900. The voice input device 966 functions, for example, as the input unit 100 described above, is provided, for example, on the information processing apparatus 900, and is connected to the input/output interface 958 in the information processing apparatus 900.

Note that it goes without saying that the input/output interface 958 can be connected to an external device such as an operation input device (for example, keyboard, mouse, and the like) outside the information processing apparatus 900 and an external display device.

Furthermore, the input/output interface 958 may be connected to a drive (not illustrated). The drive is a reader/writer for a removable recording medium such as a magnetic disk, an optical disk, or a semiconductor memory and is incorporated in the information processing apparatus 900 or attached to the outside of the information processing apparatus 900. The drive reads information recorded in the attached removable recording medium and outputs the information to the RAM 954. Furthermore, the drive can write a record to the attached removable recording medium.

### (Communication interface 968)

The communication interface 968 functions as the communication unit 340 for wiredly or wirelessly communicating with an external device such as the server 30, for example, via the above-described network 70 (or directly). Here, the communication interface 968 is, for example, a communication antenna, a radio frequency (RF) circuit (wireless communication), an IEEE 802.15.1 port and a transmitting and receiving circuit (wireless communication), an IEEE802.11 port and a transmitting and receiving circuit (wireless communication), or a local area network (LAN) terminal and transmitting and receiving circuit (wired communication).

### (Sensor 980)

The sensor 980 functions as the sensor unit 120 described above. Moreover, the sensor 980 may include various sensors such as a pressure sensor.

An exemplary hardware configuration of the information processing apparatus 900 has been described above. Note that the hardware configuration of the information processing apparatus 900 is not limited to the configuration illustrated in Fig. 34. Specifically, each component may be formed by using a general-purpose member or may be hardware specialized to the function of each component. Such a configuration may be appropriately changed according to technical level at the time of implementation.

For example, in a case of communicating with the external device via a connected external communication device and in a case of a configuration for executing processing in a stand-alone manner, the information processing apparatus 900 does not need to include the communication interface 968. Furthermore, the communication interface 968 may have a configuration which can communicate with one or two or more external devices by a plurality of communication methods. Furthermore, for example, the information processing apparatus 900 can have a configuration which does not include the recording medium 956, the operation input device 960, the display device 962, and the like.

Furthermore, the information processing apparatus 900 according to the present embodiment may be applied to, for example, a system including a plurality of devices on the premise of connection to a network (or communication between devices), for example, a cloud computing. That is, the information processing apparatus 900 according to the above-described present embodiment can be implemented, for example, as the information processing system 1 for executing the processing according to the information processing method of the present embodiment by the plurality of devices.

### <<6. Supplement>>

Note that the embodiment of the present disclosure described above may include, for example, a program for causing a computer to function as the information processing apparatus according to the present embodiment and a non-temporary tangible medium recording the program. Furthermore, the program may be distributed via a communication line such as the Internet (including wireless communication).

Furthermore, it is not necessary to process each step in the processing in each embodiment described above in an order described above. For example, each step may be processed in an appropriately changed order. Furthermore, each step may be partially processed in parallel or individually processed instead of being processed in time-series manner. Moreover, it is not necessary to execute the processing method in each step along the described method and, for example, may be processed by other method by other functional unit.

The preferred embodiment of the present disclosure has been described in detail above with reference to the drawings. However, the technical scope of the present disclosure is not limited to the example. It is obvious that a person who has normal knowledge in the technical field of the present disclosure can arrive at various variations and modifications in the scope of the technical ideas described in claims. It is understood that the variations and modifications naturally belong to the technical scope of the present disclosure.

Furthermore, the effects described in the present description are merely illustrative and exemplary and not limited. That is, the technology according to the present disclosure can exhibit other effects obvious to those skilled in the art from the description in the present description together with or instead of the above described effects.

### REFERENCE SIGNS LIST

- 1: Information processing system
- 10, 10a, 10b: Wearable device
- 12L,12R: Main body portion
- 14: Neck band
- 16: Touch panel display
- 18: Speaker
- 20: Microphone
- 30: Server
- 50, 50a: User terminal
- 52: Treadmill
- 54: Attaching gear
- 70: Network
- 90, 92: Temporal change
- 92a, 92b: Section
- 100, 300, 500: Input unit
- 110, 310, 510: Output unit
- 120: Sensor unit
- 130, 330, 530: Control unit
- 132: Learning unit
- 134: Classification unit
- 136: Estimation unit
- 140, 340, 540: Communication unit
- 150, 350: Storage unit
- 234, 238, 240: DB
- 236: Likelihood estimator
- 400, 402, 404, 406: Time-series data
- 408: Estimation likelihood
- 410: Parameter
- 420: Label
- 800, 802, 804, 806, 808, 810,:
- 812, 814, 820, 822, 824, 826, 828, 830: Screen
- 900: Information processing apparatus
- 950: CPU
- 952: ROM
- 954: RAM
- 956: Recording medium
- 958: Input/output interface
- 960: Operation input device
- 962: Display device
- 964: Voice output device
- 966: Voice input device
- 968: Communication interface
- 970: Bus
- 980: Sensor

## Claims

1. A wearable device comprising:
a sensor unit (120) including a heart rate sensor and a motion sensor, and a control unit (130), wherein
the heart rate sensor is configured to measure a heart rate of a user and output a measured result to the control unit, and wherein
the motion sensor includes at least one acceleration sensor and is configured to detect a change in acceleration caused according to an operation of the user, and output a detection result to the control unit (130);
wherein the wearable device is configured to:
acquire acceleration time-series data (400) and heart rate time-series data (402), wherein the wearable device further comprises:
a classification unit (134) configured to:
classify to which cluster of a plurality of clusters the acceleration time-series data (400) and the heart rate time-series data (402) belong based on a comparison between the heart rate time-series data with a model associated with the cluster, wherein the heart rate time series data which are similar to each other belong to the same cluster, and wherein for heart rate time series data belonging to the same cluster, consumed energy can be estimated from the heart rate time-series data by using the model as a common model; and
an estimation unit (136) configured to:
input the acceleration time-series data (400) and the heart rate time-series data (402) corresponding to the classified cluster into an estimation database (240); and
estimate consumed energy time-series data (406) on the basis of the model.

2. The wearable device according to claim 1, further comprising an instruction unit (110) configured to prompt the user to perform a predetermined exercise so as to acquire the fluctuation pattern of the beating rate of the user caused by the change in the exercise intensity.

3. The wearable device according to claim 1, further comprising a notification unit (110) configured to notify the user of the estimated consumed energy.

4. The according to claim 3, wherein the notification unit (11) is further configured to make a notification to the user for recommending the predetermined exercise to the user on a basis of the estimated consumed energy.

5. The wearable device according to claim 1, including one of a wearable terminal attached to a body of the user or an implant terminal inserted into the body of the user.

6. An information processing method comprising:
measuring a heart rate of a user, and outputting a measured result to a control unit (130);
detecting a change in acceleration caused according to an operation of the user, and outputting a detection result to the control unit (130);
acquiring acceleration time-series data (400) and heart rate time-series data (402);
classifying to which cluster of a plurality of clusters the acceleration time-series data (400) and the heart rate time-series data (402) belong based on a comparison between the heart rate time-series data with a model associated with the cluster, wherein the heart rate time series data which are similar to each other belong to the same cluster, and wherein for heart rate time series data belonging to the same cluster, consumed energy can be estimated from the heart rate time-series data by using the model as a common model;
inputting the acceleration time-series data (400) and the heart rate time-series data (402) corresponding to the classified cluster into an estimation database (240); and
estimating consumed energy time-series data (406) on the basis of the model.

7. A computer program comprising instructions which, when the computer program is carried out on the wearable device of claim 1, causes the wearable device of claim 1 to carry out the method of claim 6.

## Patentansprüche

1. Tragbare Vorrichtung, umfassend:
eine Sensoreinheit (120), die einen Herzfrequenzsensor und einen Bewegungssensor aufweist, und eine Steuereinheit (130), wobei
der Herzfrequenzsensor dafür ausgelegt ist, eine Herzfrequenz eines Benutzers zu messen und ein Messergebnis an die Steuereinheit auszugeben, und wobei der Bewegungssensor wenigstens einen Beschleunigungssensor aufweist und dafür ausgelegt ist,
eine Änderung der Beschleunigung zu erfassen, die gemäß einer Handlung des Benutzers verursacht wird, und ein Erfassungsergebnis an die Steuereinheit (130) auszugeben;
wobei die tragbare Vorrichtung ausgelegt ist zum:
Erfassen von Beschleunigungs-Zeitreihendaten (400) und Herzfrequenz-Zeitreihendaten (402), wobei die tragbare Vorrichtung ferner umfasst:
eine Klassifizierungseinheit (134), die ausgelegt ist zum:
Klassifizieren, zu welchem Cluster von mehreren Clustern die Beschleunigungs-Zeitreihendaten (400) und die Herzfrequenz-Zeitreihendaten (402) gehören, basierend auf einem Vergleich der Herzfrequenz-Zeitreihendaten mit einem dem Cluster zugeordneten Modell, wobei die Herzfrequenz-Zeitreihendaten, die einander ähnlich sind, zu demselben Cluster gehören, und wobei für Herzfrequenz-Zeitreihendaten, die zu demselben Cluster gehören, den Energieverbrauch anhand der Herzfrequenz-Zeitreihendaten geschätzt werden kann, indem das Modell als gemeinsames Modell verwendet wird; und
eine Schätzeinheit (136), die ausgelegt ist zum:
Eintragen der Beschleunigungs-Zeitreihendaten (400) und der Herzfrequenz-Zeitreihendaten (402), die dem klassifizierten Cluster entsprechen, in eine Schätzungsdatenbank (240); und
Schätzen von Energieverbrauch-Zeitreihendaten (406) basierend auf dem Modell.

2. Tragbare Vorrichtung gemäß Anspruch 1, ferner eine Anweisungseinheit (110) umfassend, die dafür ausgelegt ist den Benutzer aufzufordern, eine vorbestimmte Übung auszuführen, um das Schwankungsmuster der Herzschlagfrequenz des Benutzers, das durch die Änderung der Übungsintensität verursacht wird, zu erfassen.

3. Tragbare Vorrichtung gemäß Anspruch 1, ferner eine Benachrichtigungseinheit (110) umfassend, die dafür ausgelegt ist, den Benutzer über den geschätzten Energieverbrauch zu informieren.

4. Tragbare Vorrichtung gemäß Anspruch 3, wobei die Benachrichtigungseinheit (11) ferner dafür ausgelegt ist, eine Benachrichtigung an den Benutzer zu senden, um dem Benutzer die vorbestimmte Übung zu empfehlen, basierend auf dem geschätzten Energieverbrauch.

5. Tragbare Vorrichtung gemäß Anspruch 1, die ein am Körper des Benutzers befestigtes tragbares Endgerät oder ein in den Körper des Benutzers eingesetztes implantiertes Endgerät aufweist.

6. Informationsverarbeitungsverfahren, umfassend:
Messen der Herzfrequenz eines Benutzers und Ausgeben des Messergebnisses an eine Steuereinheit (130);
Erfassen einer Änderung der Beschleunigung, die gemäß einer Handlung des Benutzers verursacht wird, und
Ausgeben eines Erfassungsergebnisses an die Steuereinheit (130);
Erfassen von Beschleunigungs-Zeitreihendaten (400) und Herzfrequenz-Zeitreihendaten (402);
Klassifizieren, zu welchem Cluster von mehreren Clustern die Beschleunigungs-Zeitreihendaten (400) und die Herzfrequenz-Zeitreihendaten (402) gehören, basierend auf einem Vergleich der Herzfrequenz-Zeitreihendaten mit einem dem Cluster zugeordneten Modell, wobei die Herzfrequenz-Zeitreihendaten, die einander ähnlich sind, zu demselben Cluster gehören, und wobei für Herzfrequenz-Zeitreihendaten, die zu demselben Cluster gehören, den Energieverbrauch anhand der Herzfrequenz-Zeitreihendaten geschätzt werden kann, indem das Modell als gemeinsames Modell verwendet wird;
Eintragen der Beschleunigungs-Zeitreihendaten (400) und der Herzfrequenz-Zeitreihendaten (402), die dem klassifizierten Cluster entsprechen, in eine Schätzungsdatenbank (240); und
Schätzen von Energieverbrauch-Zeitreihendaten (406) basierend auf dem Modell.

7. Computerprogramm, das Anweisungen umfasst, die, wenn das Computerprogramm auf der tragbaren Vorrichtung nach Anspruch 1 ausgeführt wird, die tragbare Vorrichtung nach Anspruch 1 veranlassen, das Verfahren nach Anspruch 6 auszuführen.

## Revendications

1. Dispositif portable comprenant :
une unité de capteur (120) comprenant un capteur de fréquence cardiaque et un capteur de mouvement, et une unité de commande (130),
le capteur de fréquence cardiaque étant configuré pour mesurer la fréquence cardiaque d'un utilisateur et transmettre un résultat mesuré à l'unité de commande, et
le capteur de mouvement comprenant au moins un capteur d'accélération et étant configuré pour détecter un changement d'accélération provoqué en fonction d'une opération de l'utilisateur, et transmettre un résultat de détection à l'unité de commande (130) ;
le dispositif portable étant configuré pour :
acquérir des données de séries temporelles d'accélération (400) et des données de séries temporelles de fréquence cardiaque (402), le dispositif portable comprenant en outre :
une unité de classification (134) configurée pour :
classer à quel groupe d'une pluralité de groupes les données de séries temporelles d'accélération (400) et les données de séries temporelles de fréquence cardiaque (402) appartiennent sur la base d'une comparaison entre les données de séries temporelles de fréquence cardiaque avec un modèle associé au groupe, les données de séries temporelles de fréquence cardiaque qui sont similaires les unes aux autres appartenant au même groupe, et pour les données de séries temporelles de fréquence cardiaque appartenant au même groupe, l'énergie consommée pouvant être estimée à partir des données de séries temporelles de fréquence cardiaque en utilisant le modèle comme modèle commun ; et
une unité d'estimation (136) configurée pour :
entrer les données de séries temporelles d'accélération (400) et les données de séries temporelles de fréquence cardiaque (402) correspondant au groupe classé dans une base de données d'estimation (240) ; et
estimer les données de série temporelle d'énergie consommée (406) sur la base du modèle.

2. Dispositif portable selon la revendication 1, comprenant en outre une unité d'instruction (110) configurée pour inviter l'utilisateur à réaliser un exercice prédéterminé de manière à acquérir le modèle de fluctuation de la fréquence cardiaque de l'utilisateur causé par le changement de l'intensité de l'exercice.

3. Dispositif portable selon la revendication 1, comprenant en outre une unité de notification (110) configurée pour notifier à l'utilisateur l'énergie consommée estimée.

4. Dispositif portable selon la revendication 3, l'unité de notification (11) étant en outre configurée pour faire une notification à l'utilisateur pour recommander l'exercice prédéterminé à l'utilisateur sur la base de l'énergie consommée estimée.

5. Dispositif portable selon la revendication 1, comprenant l'un d'un terminal portable fixé à un corps de l'utilisateur ou d'un terminal d'implant inséré dans le corps de l'utilisateur.

6. Procédé de traitement d'informations comprenant :
la mesure de la fréquence cardiaque d'un utilisateur, et la transmission d'un résultat mesuré à une unité de commande (130) ;
la détection d'un changement d'accélération causé par une opération de l'utilisateur, et la transmission d'un résultat de détection à l'unité de commande (130) ;
l'acquisition de données de séries temporelles d'accélération (400) et de données de séries temporelles de fréquence cardiaque (402) ;
le classement à quel groupe d'une pluralité de groupes les données de séries temporelles d'accélération (400) et les données de séries temporelles de fréquence cardiaque (402) appartiennent sur la base d'une comparaison entre les données de séries temporelles de fréquence cardiaque avec un modèle associé au groupe, les données de séries temporelles de fréquence cardiaque qui sont similaires les unes aux autres appartenant au même groupe, et pour les données de séries temporelles de fréquence cardiaque appartenant au même groupe, l'énergie consommée pouvant être estimée à partir des données de séries temporelles de fréquence cardiaque en utilisant le modèle comme modèle commun ;
l'entrée des données de séries temporelles d'accélération (400) et des données de séries temporelles de fréquence cardiaque (402) correspondant au groupe classé dans une base de données d'estimation (240) ; et
l'estimation de données de série temporelle d'énergie consommée (406) sur la base du modèle.

7. Programme informatique comprenant des instructions qui, lorsque le programme informatique est exécuté sur le dispositif portable selon la revendication 1, amènent le dispositif portable selon la revendication 1 à réaliser le procédé selon la revendication 6.
